(19)

Europäisches
Patentamt
European
Patent Office
Office européen
des brevets

(11) **EP 4 364 789 B1**

(12) **EUROPEAN PATENT SPECIFICATION**

(45) Date of publication and mention
of the grant of the patent:
**22.04.2026  Bulletin 2026/17**

(21) Application number: **21946676.0**

(22) Date of filing: **10.08.2021**

(51) International Patent Classification (IPC):
**A61N 1/36** (2006.01)      **A61N 1/05** (2006.01)

(52) Cooperative Patent Classification (CPC):
**A61N 1/36125; A61N 1/0502; A61N 1/0551;
A61N 1/0558; A61N 1/3605**

(86) International application number:
**PCT/CN2021/111817**

(87) International publication number:
**WO 2022/267191 (29.12.2022 Gazette 2022/52)**

(54) **PERIPHERAL NERVE STIMULATION SYSTEM**

SYSTEM ZUR PERIPHEREN NERVENSTIMULATION

SYSTÈME DE STIMULATION NERVEUSE PÉRIPHÉRIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.06.2021  CN 202110701753**

(43) Date of publication of application:
**08.05.2024  Bulletin 2024/19**

(73) Proprietor: **Jiangsu Ced Medtech Co., Ltd
Rudong Nantong, Jiangsu 226499 (CN)**

(72) Inventors:
 • **YE, Jinnan
   Shanghai 201210 (CN)**
 • **HUANG, Baoming
   Shanghai 201210 (CN)**

 • **ZHU, Yueliang
   Shanghai 201210 (CN)**
 • **ZHANG, Shan
   Shanghai 201210 (CN)**

(74) Representative: **Loo, Chi Ching et al
Albright IP Limited
County House
Bayshill Road
Cheltenham, Gloucestershire GL50 3BA (GB)**

(56) References cited:
**EP-A1- 1 005 879      CN-A- 108 472 484
CN-U- 206 120 952      US-A- 5 366 493
US-A1- 2004 111 139      US-A1- 2008 027 524
US-A1- 2008 071 321      US-A1- 2010 152 808
US-A1- 2017 340 891      US-A1- 2018 056 066**

## Description

## Technical Field

The present invention relates to the field of medical devices, and more particularly to a peripheral nerve stimulation system.

## Background Art

It is well known that pain is a difficult disease to cure in humans, that the mechanism of pain is extremely complex. The electrical stimulation therapy has a long history, including implantable spinal cord stimulators, non-implantable epidermal stimulation, and peripheral nerve therapy with percutaneous implanted leads and external electrical stimulators. Although these devices reduce pain and improve the quality of life for patients, these stimulation systems have various defects and deficiencies, as well as differences in the patient's human anatomy, insufficient electrical stimulation energy (including electrode displacement), inability of electrical stimulation to cover the target of painful nerve, etc., resulting in ineffective pain suppression in patients.

Patent No. US4556051, filed in 1985, and Patent No. US5830151, filed in 1998, disclose methods and devices for the treatment of peripheral nerve electrical stimulation. Although the treatment of peripheral nerve electrical stimulation has a long history, it is not widely used because of the poor effectiveness of surface electrodes to provide electrical stimulation and the inconvenience of wearing electrical stimulators which are relatively large.

Patent No. US20180056066A1 discloses a percutaneous stimulation system in which a loop is created between a percutaneous electrode and a surface electrode so as to achieve the effect of nerve electric stimulation. However, the surface electrode in the system can only be below the stimulator. If the surface electrode is on the surface of the human body where the stimulator is inconveniently placed, the surface electrode cannot be realized, thus reducing the range of surface electrode that can be placed. The optimal path for stimulation may not be reached, resulting in that the effect of treating nerve pain for patients is limited. Most importantly, the above patent has only a single percutaneous electrode, which can only achieve monopolar stimulation of a percutaneous electrode and stimulation of a single path. The effect of peripheral nerve stimulation is influenced by the relative position of the stimulation electrode and the target nerve. Stimulation may only occur if the target nerve is in the stimulation path. Also, if the electrode is close to the target nerve, the stimulation current easily covers the target nerve, thereby easily producing a better effect, and conversely, hardly having an effect. Therefore, the stimulation effect of the single electrode is easily influenced by the implantation position. Even if the electrode is in place at the time of implantation, the percutaneous implanted lead may dislodge over time. Additionally, a single percutaneous electrodeis is not capable of bipolar or multipolar stimulation in vivo. All of the above affect the effectiveness of the single percutaneous implanted lead system in stimulating nerves and treating pain.

At present, many doctors implant spinal stimulation electrodes into peripheral nerve for pain treatment. However, the percutaneous implanted lead for spinal cord stimulation adopts the closed insulating layer which is disposed on the whole periphery after the metal wire is spirally wound. Its diameter is generallybig, ranging from 1.3 mm-1.4 mm. Therefore, the outer diameter of the needle tube of the puncture needle in which the percutaneous implanted lead is required to be implanted is also relatively bigger. It is difficult to implant it into the peripheral nerve, causing great trauma to patients and increasing the risk of infection. The clinical need for peripheral nerves is to design the percutaneous implanted lead to be 0.70 mm with smaller diameter. The design and manufacture of the percutaneous implanted lead in this size range has great challenges and difficulties. We face the problem how to design the spiral conductor wire under the limited cross-sectional area and resist fatigue distortion and bending, and the insulation between spiral conductor wires is also a great challenge.

In addition, Patent No. US20180056066A1 discloses a percutaneous stimulation system in which the output of the stimulation is a square wave current pulse of fixed amplitude and frequency, the parameters of which are fixed as soon as the physician selects a stimulation procedure. The pulse waveform template is a minimum stimulation unit, which is composed of a stimulation phase pulse and an equilibrium phase pulse, and is simply called stimulation pulse. Studies have shown that different nerve fibers have different response spectrum to stimulation. The spectrum of stimulation pulse also affects the propagation distance of stimulation current. For example, a slowly rising pulse current can affect deeper nerves. The effect of sparse and dense wave pulse sequence with alternating output of low frequency and high frequency is better than that of single frequency, etc. The stimulators of the above-mentioned patents, as well as most existing stimulators, can only output a single-frequency square-wave voltage or current stimulation pulse waveform with a fixed rising edge. They lack the possibility of selecting the target nerve and thus have corresponding limitations for pain suppression. Furthermore, the stimulation with a constant amplitude and frequency can easily cause fatigue and habituation in the nervous system, thereby affecting the therapeutic effect.

Patent No. US 2010/152808 A1 discloses a method to reduce pain by stimulating a spinal nerve of passage innervating a painful region with an intramuscular (percutaneous) lead inserted into muscle tissue near the nerve of passage and upstream of the painful region.

## Summary of the Invention

The technical problem to be solved by the present invention is to provide a peripheral nerve stimulation system, which may effectively inhibit pain and improve clinical therapeutic effect.

The present invention is set forth in the appended set of claims.

The present invention has the following advantageous effects compared to the prior art. According to the peripheral nerve stimulation system provided by the present invention, the lead body of the percutaneous implanted lead forms an open spiral structure after the metal wire being spirally wound and insulated, which is more conducive to the lead body to bear the traction of the muscle tissue in the body and has a better elasticity than the case where is forms a closed insulating layer around the whole periphery after the metal wire being spirally wound. Due to the open spiral structure of the lead body, the diameter of the percutaneous part of the lead body may be reduced to the diameter of a single wire plus the thickness of the wrapped insulating layer (0.20 mm-0.30 mm), rather than the diameter (0.50 mm-0.70 mm) formed by wrapping the insulating layer integrally after the spiral winding, which has less trauma and faster healing and reduces the risk of infection. In addition, it may be integrally wound with one or two metal wires with the insulating layer, so as to reduce the risk of subsequent fracture caused by processing technology in the connection process of electrical conductor and the risk of connection separation caused by fatigue and wear in clinical use. In particular, the tightly wound spiral electrode structure may increase the stimulation surface area for human body and improve the bearable stimulation current. In particular, the lead body of the percutaneous implanted lead is designed by using extremely fine double-stranded insulated metal wire in a stacked spiral shape, and a cylindrical or stacked spiral double-electrode structure and a structure fixed to the human tissue are formed at the distal end of the percutaneous implanted lead. The three electrodes composed of internal double electrodes and a surface electrode may form six different stimulation pathways composed of stimulation electrodes and return electrodes, and each stimulation pathway may play a role in different nerve tissues. Furthermore, in the case of a combination of two electrodes, by distributing the current intensity between the two electrodes, the overall flow direction of the current is controlled, reaching the effect of the virtual electrode, providing a new possibility to increase the stimulation effect. In addition, the surface electrodes may be placed not only directly on the bottom of the stimulator, but also connected to the electrode interfaces on the bottom of the stimulator by the cables, so that they can be placed anywhere the patient needs stimulation, for example, where they are particularly sensitive to stimulation. In particular, a micro-adapter is provided between the percutaneous implanted lead and the stimulator to protect the percutaneous implanted lead from the skin wound and transmit the stimulation signal, thereby reducing the chance of accidental wound injury and infection during treatment.

## Brief Description of the Drawings

Fig. 1 is a schematic view showing an overall structure of a peripheral nerve stimulation system according to an example of the present invention;

Figs. 2a and 2b are schematic views showing the structure of a remote controller according to an example of the present invention, and Fig. 2c is a schematic view of a stimulation program controller;

Fig. 3a is a schematic view showing the structure of a fixed surface electrode according to an example of the present invention, and Fig. 3b is a schematic view showing the structure of a movable surface electrode;

Figs. 4a and 4b are schematic views showing the structure of a percutaneous implanted lead having a monopolar electrode according to a first example of the present invention;

Fig. 5 is a schematic view showing the structure of a percutaneous implanted lead having a monopolar electrode according to a second example of the present invention;

Fig. 6 is a schematic view showing the structure of a percutaneous implanted lead having a bipolar electrode according to a third example of the present invention;

Fig. 7 is a schematic view showing the structure of a percutaneous implanted lead having a bipolar electrode according to a fourth example of the present invention;

Fig. 8a is a schematic view showing an overall structure of an adapter mounted on a mounting seat according to an example of the present invention, and Fig. 8b is an exploded schematic view of Fig. 4a;

Fig. 8c is a schematic view of an overall structure of the adapter; Fig. 8d is a schematic view of the structure of a second housing, and Fig. 8e is a schematic view of an overall structure of the mounting seat;

Fig. 9 is a schematic view of a peripheral nerve stimulation system for a patient according to an example of the present invention;

Fig. 10 is a schematic circuit diagram of a stimulator according to an example of the present invention;

Fig. 11(a) is a schematic view of six possible stimulation paths of three electrodes, and Fig. 11(b) is a schematic view of a virtual electrode formed by different division of two electrode currents of an electrode combination and a change in the direction of the total current;

Fig. 12(a) is a schematic view of a stimulation loop formed by a stimulation electrode and a return electrode , and Fig. 12(b) is a schematic view of the operating principle of a bi-directional current pulse source;

Fig. 13 is a schematic circuit diagram of a stimulator employing a single unidirectional current pulse source according to another example of the present invention;

Fig. 14(a) is a schematic view of a measurement circuit structure of a stimulator according to an example of the present invention; Fig. 14(b) is a schematic view showing a neural response signal measurement procedure according to an example of the present invention;

Fig. 15 is a schematic view of five stimulation pulse waveform templates according to an example of the present invention;

Fig. 16(a) is a schematic view of a constant-frequency constant-amplitude continuous stimulation mode and a burst stimulation mode according to an example of the present invention; Fig. 16(b) is a schematic view of a dual-frequency pulse continuous stimulation mode and a burst stimulation mode according to an example of the present invention;

Fig. 17 is a schematic view of a simulated amplitude modulation stimulation mode according to an example of the present invention, the modulation manner including sinusoidal, triangular, and sawtooth modulation;

Fig. 18 is a schematic view of a simulated frequency modulated stimulation pattern according to an example of the present invention, the modulation manner including sine wave, triangular wave, and sawtooth wave modulation;

Fig. 19 is a schematic view of a keying-shift modulation stimulation mode according to an example of the present invention, the modulation manner including amplitude shift keying, frequency shift keying, and a combination of amplitude shift keying and frequency shift keying;

Fig. 20 is a schematic view of a method for implementing stimulation modulation by a stimulation controller according to an example of the present invention;

Fig. 21 is a flow diagram showing the implementation of patient tuning settings for a stimulation prescription according to an example of the present invention;

Fig. 22 is a schematic view of a programming interface of a physician's stimulation program controller according to an example of the present invention.

In the drawings,
1-subcutaneous tissue, 2-wound, 3-patient, 10-percutaneous implanted lead, 11-distal lead head, 12-lead body, 13-proximal lead end, 20-adapter, 21-mounting seat, 211-base, 212-main body portion, 2121-top, 2122-arch strip, 30-first cable, 31-first wiring port, 40-third cable, 41-second wiring port, 50-stimulator, 51-stimulation controller, 52-pulse generator, 521-pulse source, 53-third wiring port, 60-remote controller, 61-control key, 62-display screen, 63-hanging hole, 64-hand grip, 65-USB interface, 70-fixed surface electrode, 701-first surface patch, 801-second surface patch, 702, 802-hydrogel, 71-first surface electrode interface, 72-second cable, 80-movable surface electrode, 81-second surface electrode interface, 91-third surface electrode interface, 901-third surface patch, 902-adhesive, 100-clinical programmer, 111-first electrode, 112-second electrode, 113-fixed hook, 114-insulating buffer sleeve, 121-conductor wire, 122-metal wire, 123-insulating layer, 1131-bent portion, 1132-linear portion, 1211-first conductor wire, 1212-second conductor wire, 2111-first guide rail, 2112-second guide rail, 2113-limiting piece, 2114-first snap, 201-first housing, 202-second housing, 203-first channel, 204-second channel, 205-through hole, 2021-first side wing, 2022-second side wing, 2031-first flexure strip, 2032-second flexure strip, 2033-first blade, 2034-second blade, 2041-third flexure strip, 2042-fourth flexure strip, 2043-third blade, 2044-fourth blade, 2023-interface, 2024-first protrusion, 2025-second protrusion, 2011-first slot, 2012-second slot.

**Detailed Description of the Invention**

The present invention are further described below in combination with the attached drawings and examples. In order to more clearly describe the structural features of the present invention, the present invention uses "proximal", "distal", and "axial" as directional terms, wherein "proximal" refers to an end near to the operator; "distal" refers to an end away from the operator; "distal lead head" refers to a most distal part of the percutaneous implanted lead; and "axial" refers to a direction of the central axis of the electrode or a direction parallel to the central axis of the electrode. The term "or" is generally employed in its sense including "and/or" unless the content clearly dictates otherwise.

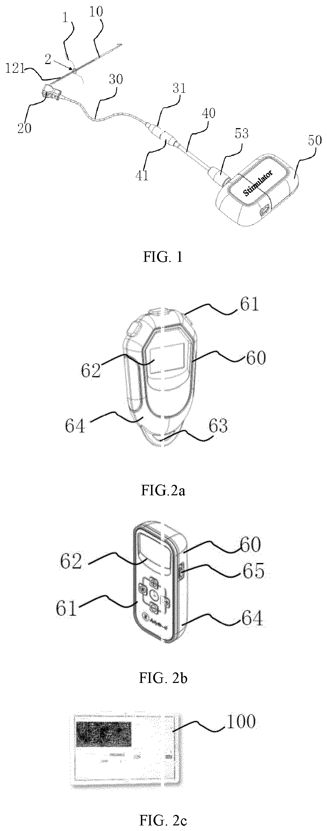

Referring to Figs. 1, 2a-2c, 3a and 3b, the present example provides a peripheral nerve stimulation system including an percutaneous implanted lead 10, an adapter 20, a first cable 30, a stimulator 50, a remote controller 60 and a surface electrode. The percutaneous implanted lead 10 may be percutaneously inserted into a patient body. The percutaneous implanted lead 10 includes an distal lead head 11 and an lead body 12 in sequence from the distal end to the proximal end. The distal lead head 11 includes at least one electrode. The lead body 12 is formed by spirally winding at least one conductor wire 121. The conductor wire 121 includes a metal wire 122 and an insulating layer 123. The metal wire 122 surrounds the insulating layer 123, so that the lead body 12 is of an open insulating spiral structure. The spiral turns constituting the lead body 12 are sequentially arranged at a predetermined pitch along the axial direction. It is advantageous for the lead body 12 to withstand the pulling of the muscle tissue in the body, to have a more excellent elasticity, and to have a pitch size specially designed as required, preferably 0.38 mm-0.48 mm. The metal wire 122 is preferably a 316L stainless steel wire or a MP35N non-magnetic nickel-cobalt-chromium-molybdenum alloy wire. The insulating material of the insulating layer 123 is preferably polyurethane (PU), polytetrafluoro ethylene (PTFE) or ethylene-tetrafluoro ethylene copolymer (ETFE). The insulating material is extruded into a tube type and then applied to the periphery of the metal wire 122, or the insulating material is sprayed onto the metal wire 122 by using a coating process. The metal wire 122 may be a very fine metal wire. The diameter of the metal wire is preferably 0.1 mm -0.15 mm, and the diameter of the prepared percutaneous percutaneous implanted lead is 0.55 mm-0.70 mm, which can meet the clinical requirements of peripheral nerve stimulation. Therefore, compared with the prior art in which the metal wire is spiraled to form a closed insulation structure at the periphery of the whole spiral structure, due to the open spiral structure of the lead body 12, the diameter of the percutaneous portion of the lead body 12 is reduced to a single wire diameter plus the thickness of the wrapped insulating layer (0.20 mm-0.30 mm), rather than the diameter formed after the the spiral winding (0.50 mm-0.70 mm), after contraction and compression of the skin wound, which results in less trauma and faster healing for the patient, reduces the risk of wound infection and provides more effective treatment for neuromodulation.

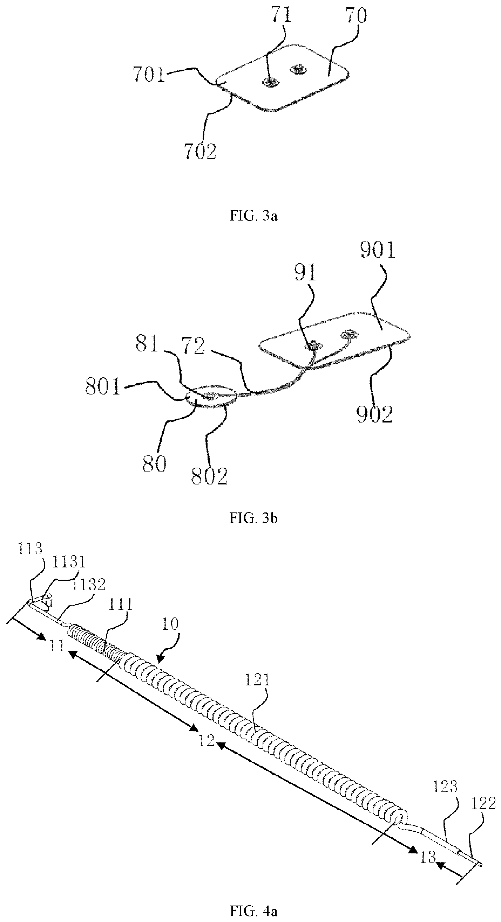

With continued reference to Fig. 1, the adapter 20 is electrically connected to the proximal end of the percutaneous implanted lead 10. Further, the conductor wire 121 constituting the lead body 12 may be linearly extended towards the proximal end to form a proximal lead end 13. The adapter 20 is electrically connected to the metal wire 122 constituting the lead body 12. The adapter 20 may establish a reliable connection with the inner metal wire 122 after penetrating the insulating layer 123 of the conductor wire 121. The connection position may be on the lead body 12 or on the proximal lead end 13. In other embodiments, the metal wire 122 at the distal end of the proximal lead end 13 may be exposed and electrically connected to the adapter 20. The adapter 20 is electrically connected to the stimulator 50 via the first cable 30, and the stimulator 50 is used for sending electrical stimulation pulses. The surface electrode is electrically connected to the stimulator 50 and forms a loop with the percutaneous implanted lead 10. As shown in Fig. 3a, in an embodiment, the surface electrode is a fixed surface electrode 70 which is fixed on the bottom of the stimulator 50 and is electrically connected to the stimulator 50 via a first surface electrode interface 71. Specifically, the fixed surface electrode 70 is composed of a first surface patch 701, a hydrogel 702 and the first surface electrode interface 71. One side of the first surface patch 701 is connected to the stimulator 50. The hydrogel 702 is coated on the first surface patch 701 so as to adhere the whole stimulator 50 to the human skin. The first surface electrode interface 71 is disposed on the first surface patch 701 and is in communication with the hydrogel 702, which is conductive to form a conductive layer. Meanwhile, the stimulator 50 is provided with an electrode interface. The fixed surface electrode 70 and the stimulator 50 are electrically and mechanically connected via the surface electrode interface 71 and the electrode interface on the stimulator 50, so that the first surface patch 701 is integrated with the stimulator 50. Then the whole stimulator 50 is pasted and fixed on the body surface of the patient via the hydrogel 702 on the surface thereof. The stimulator 50, the percutaneous implanted lead 10 and the hydrogel 702 form a loop in the human body to constitute a pathway for achieving electrical stimulation. Alternatively, in another embodiment, as shown in Fig. 3b, the surface electrode is a movable surface electrode 80 including a second surface patch 801, a second surface electrode interface 81 and a hydrogel 802. The hydrogel 802 is coated on the second surface patch 801 to enable the movable surface electrode 80 to be attached to the human skin. The second surface electrode interface 81 is disposed on the second surface patch 801 and is in communication with the hydrogel 802. The hydrogel 802 forms a conductive layer. In this embodiment, it further includes a third surface patch 901. A third surface electrode interface 91 is disposed on the third surface patch 901. The third surface patch 901 is coated with an adhesive 902 which is a skin-friendly adhesive and is non-conductive. The whole stimulator 50 is adhered to the human skin via the adhesive 902. A patient or a doctor may choose to connect the movable surface electrode 80 with the stimulator 50 via the second cable 72. Specifically, one end of the second cable 72 is connected with the second surface electrode interface 81, and the other end of the second cable 72 is connected with the third surface electrode interface 91. The third surface electrode interface 91 is electrically and mechanically connected with the electrode interface on the stimulator 50, so that the third surface patch 901 is integrated with the stimulator 50. Then, the whole stimulator 50 is pasted and fixed on the patient's body surface by the adhesive 902 on the surface thereof. The stimulator 50, the percutaneous implanted lead 10 and the hydrogel 802 form a loop in the human body, constituting a pathway for realizing electrical stimulation. The position of the movable surface electrode 80 may be freely moved, so that the electrical performance is optimal. By exiting the second cable 72, the movable surface electrode 80 may be placed anywhere the patient requires stimulation, such as where it is most sensitive to stimulation, so as to more effectively suppress pain. Further, it also includes a third cable 40, the first cable 30 and the third cable 40 being detachably connected, and the third cable 40 being detachably connected to the stimulator 50. Specifically, the proximal end of the first cable 30 has a first connection port 31. The distal end of the third cable 40 has a second connection port 41. The first connection port 31 and the second connection port 41 are matched with the detachable connection. The proximal end of the third cable 40 has a third connection port 53, the third connection port 53 being detachably connected to the stimulator 50 for convenient use. In other embodiments, the first cable 30 and the third cable 40 are non-removably and integrally connected. Whether the cables are detachably connected or not is set as needed, and the present invention is not limited thereto.

The specific working process and principle of the peripheral nerve stimulation system of the present invention are as follows. After the distal end of the tiny percutaneous implanted lead 10 is implanted near the peripheral nerve related to pain suppression, the proximal end of the percutaneous implanted lead 10 exposed outside the human epidermis is connected to the stimulator 50 via the micro-adapter 20. The stimulator 50 forms a loop with the percutaneous implanted lead 10 by the fixed surface electrode 70 or the movable surface electrode 80. The stimulator 50 sends an electrical stimulation pulse required for treatment, so as to achieve the purpose of nerve regulation. The stimulation pulse may be controlled by the remote controller 60 to select different pre-set stimulation parameters, such as pulse width, frequency and amplitude. The percutaneous implanted lead 10 is a bipolar electrode, which may further improve the clinical therapeutic effect achieved by current unipolar electrode stimulation.

With continued reference to Figs. 2a, 2b and 2c, the patient selects the stimulation program and stimulation intensity prescribed by the physician via a remote controller 60 which is in wireless communication with the stimulator 50. Specifically, in an embodiment, as shown in Fig. 2a, the remote controller 60 includes a hand grip 64, preferably sized and shaped to allow the patient to grasp with one hand, a control key 61 disposed at an end of the hand grip 64 to facilitate one-handed operation of the patient, a display screen 62 and a hanging hole 63. The hanging hole 63 is disposed at a tail portion of the hand grip and is hung and stored when the patient is not in use. The display screen 62 is provided at the top surface of the hand grip 64 for displaying stimulation parameters and the like. The remote controller 60 stores a stimulation program sequence number and a stimulation intensity sequence number corresponding to the contents of a program table and an intensity table in a hardened memory of the stimulator 50, respectively. In another embodiment, as shown in Fig. 2b, the remote controller 60 includes a hand grip 64, a control key 61 and a display screen 62. The size and shape of the hand grip 64 are preferably able to be gripped by a single hand of a patient. The control key 61 and the display screen 62 are both provided on the top surface of the hand grip 64 for the remote control operation of a single hand of a patient. Further, the side surface of the hand grip 64 is provided with a USB interface 65 for charging the remote controller 60. Further, as shown in Fig. 2c, the stimulator 50 may be controlled by the clinical programmer 100, which sets stimulation parameters and stimulation programs for the stimulator 50 by installing a stimulation programming application program on a mobile terminal, which may be a mobile phone, a tablet computer, etc.

Referring to Fig. 4a, in an example, the percutaneous implanted lead 10 has an electrode in a monopolar electrode structure. The distal lead head 11 includes a first electrode 111 and a fixation hook 113. The lead body 12 is formed by spirally winding an conductor wire 121. The spacing between adjacent spiral turns constituting the lead body 12 is preferably 0.35 mm-0.45 mm. The first electrode 111 is formed into a spiral structure by tightly winding the metal wire 122 constituting the conductor wire 121 in a distal direction under an exposed state. The spiral turns constituting the first electrode 111 are successively fitted along the axial direction. The tightly wound structure of the first electrode 111 may increase the stimulation surface area of the percutaneous implanted lead 10. The metal wire 122 at the distal end of the first electrode 111 continues to linearly extend to the distal end and is bent to form the fixation hook 113. The bending angle $\alpha$ formed between the bent portion 1131 of the fixation hook 113 and the linear portion 1132 is 10°-60°, preferably 20°-60°. The fixation hook 113 is provided so as to maintain the stimulation position of the first electrode 111 during the treatment of the patient (30-90 days or longer), and reduce the dislocation of the first electrode 111 so as to improve the treatment effect. The lead body 12 may also be implanted in a patient, and the flexibility resulting from arranging the lead body 12 in a special pitch and open spiral structure may overcome muscle stretching and twisting in the patient. The open insulated spiral structure in turn may reduce the diameter of the percutaneous electrode 10 to an extreme value, effectively reducing the risk of wound infection. The percutaneous implanted lead 10 with this structure may be integrally processed as a whole. An insulating layer 123 is formed outside the metal wire 122 where insulation is required by the coating processing technology, so that it has a small diameter and high strength. Then, the distal lead head 11, the lead body 12 and the proximal lead end 13 are integrally wound by the spiral winding process, which greatly reduces the risk of subsequent fracture caused by the processing technology during the connection of electrical conductors and the risk of connection separation in clinical use.

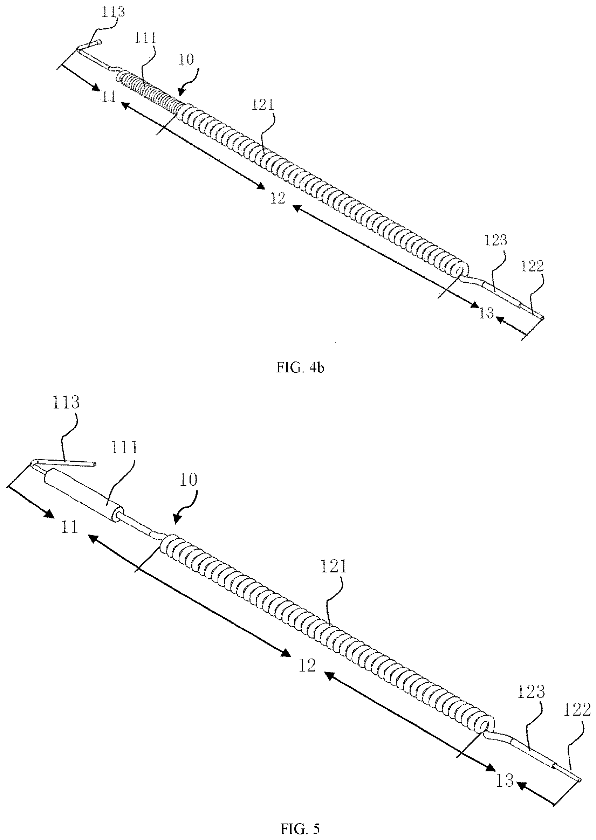

Further, as shown in Fig. 4b, in this embodiment, the pitch of the first electrode 111 between adjacent spiral coils within 2-3 turns of the distal end is greater than elsewhere. This structure is similar to that shown in Fig. 4a, but the addition of a 1-2 turn large pitch transition between the first electrode 111 and the fixation hook 113 reduces the stress concentration at this location, thereby improving the overall tensile strength at the location of the fixation hook 113.

With reference to Fig. 5, in another example, the percutaneous implanted lead 10 has one electrode, which is a monopolar electrode structure. The distal lead head 11 includes a first electrode 111 and a fixation hook 113. The first electrode 111 is a tubular metal electrode. The metal electrode may be a platinum-iridium electrode which is a good percutaneous implanted lead body and has better electricity and in-vivo corrosion resistance than a general metal wire. The lead body 12 is formed by spirally winding an conductor wire 121. In an embodiment, the metal wire 122 constituting the conductor wire 121 extends distally through an electrode tube of the metal electrode under an exposed state and then continues to extend and bend to form the fixation hook 113, and the metal wire 122 is electrically connected to the metal electrode. It directly uses a tubular metal electrode as the first electrode 111 in the structure, without performing a winding process requiring a higher consistency for the first electrode 111. The processing technology is relatively simple, and the first electrode 111 of the tubular structure has a stronger tensile resistance structure than that of the spiral structure. In another embodiment, the distal end of the metal wire 122 constituting the conductor wire 121 extends into the proximal end behind the electrode tube of the metal electrode and is electrically connected to the first electrode 111. The fixation hook 113 is made of another metal wire or other materials. The material of the fixation hook 113 may be the same as that of the metal wire 122, may also be a material different from that of the metal wire 122 according to needs, and may even be a non-metallic insulating material. Whether the fixation hook 113 and the first electrode 111 are selected is based on the electrical stimulation requirements. The proximal end of the fixation hook 113 extends into the distal end of the electrode tube of the metal electrode and is connected to the first electrode 111. This structure may change the material and structure of the fixation hook 113 more flexibly, thereby achieving a better fixing effect. The metal wire 122 establishes a reliable mechanical connection or a mechanical and electrical simultaneous connection with the first electrode 111 by welding, crimping or other mechanical connection means. The crimping method refers to a connecting method in which a portion of one part is inserted into another part and then both parts are fixed by pressing the combined portion. The crimping method in the present example means that the exposed metal wire 122 of the conductor wire 121 or the metal wire constituting the fixation hook 113 is inserted into the electrode tube, and then the electrode tube is squeezed to establish a reliable electrical connection between the metal wire 122 and the electrode tube.

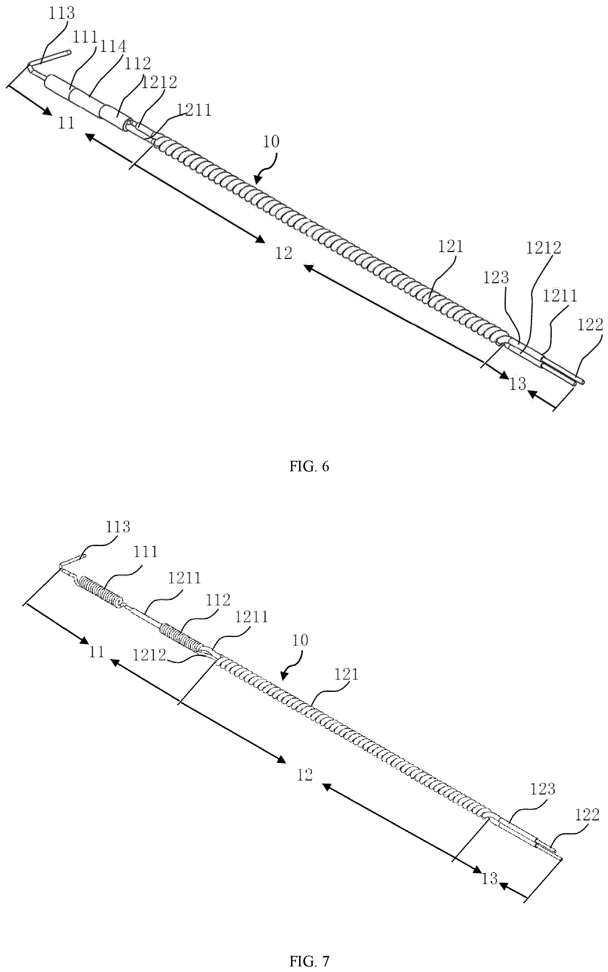

With reference to Figs. 6 and 7, in another example, the percutaneous implanted lead 10 has two electrodes in a double-electrode structure. The distal lead head 11 includes, in sequence from the proximal end to the distal end, a second electrode 112, a first electrode 111 and a fixation hook 113. The first electrode 111 and the second electrode 112 are spaced apart and insulated along the same axial direction, and the spacing distance is preferably 10-15 mm. The lead body 12 is formed by spirally winding two conductor wires 121. The two conductor wires 121 are a first conductor wire 1211 and a second conductor wire 1212, respectively. The first electrode 111 is connected to the adapter 20 via the first conductor wire 1211. The second electrode 112 is connected to the adapter 20 via the second conductor wire 1212, any one electrode or a combination of any two electrodes of the first electrode 111, the second electrode 112 and the surface electrode 70 serves as a stimulation electrode or a return electrode , and constitutes a stimulation return together with the remaining one or two electrodes. Therefore, the three electrodes of the first electrode 111, the second electrode 112 and the surface electrode may form six different stimulation pathways composed of a stimulation electrode and a return electrode . Each stimulation pathway may have an effect on different nerve tissues, or six stimulation effects may be obtained under the same electrode implantation conditions. Furthermore, in the case of a combination of two electrodes, by distributing the current intensity between the two electrodes, the overall flow direction of the current is controlled, reaching the effect of the virtual electrode, providing a new possibility to increase the stimulation effect. Compared with the unipolar structure, the percutaneous implanted lead 10 with the bipolar structure has larger stimulation range and more stimulation modes, increases the diversity of treatment and realizes different stimulation functions and stimulation effects, which is beneficial to improve the clinical treatment effect.

With continuing reference to Fig. 6, in a specific embodiment, both the first electrode 111 and the second electrode 112 are tubular metal electrodes. The metal electrodes may be platinum-iridium electrodes. The first conductor wire 1211 passes through the electrode tube of the second electrode 112. The metal wire 122 constituting the first conductor wire 1211 extends distally through the electrode tube of the first electrode 111 under an exposed state and then continues to extend distally and is bent to form the fixation hook 113. The exposed metal wire 122 is electrically connected to the first electrode 111. The exposed wire 122 at the distal end of the second conductor wire 1212 extends into the electrode tube of the second electrode 112 and is electrically connected to the second electrode 112. Further, the first conductor wire 1211 between the first electrode 111 and the second electrode 112 is provided with an insulating buffer sleeve 114 to prevent a short circuit between the first electrode 111 and the second electrode 112 and improve electrical safety. In another specific embodiment, referring to Fig. 7, the second electrode 112 is formed by the metal wire 122 constituting the second conductor wire 1212 extending distally and spirally wound under an exposed state. The distal end of the first conductor wire 1211 passes through a cavity formed spirally by the second electrode 112. The first electrode 111 is formed by the metal wire 122 constituting the first conductor wire 1211 extending distally and spirally wound under an exposed state. The metal wire 122 at the distal end of the first electrode 111 continues to extend distally and is bent to form the fixation hook 113. The percutaneous implanted lead 10 of this structure is formed by winding two wires 122 with insulating layers 123, and two conductor wires 121 are alternately wound into one along the same central axis. The first electrode 111 is formed by tightly winding the metal wire 122 with the insulating layer being removed from the distal end of the first conductor wire 1211 or the directly exposed metal wire 122. The second electrode 112 formed by tightly winding the metal wire 122 with the insulating layer 123 being removed from the distal end of the second conductor wire 1212 or the directly exposed metal wire 122 on the first conductor wire 1211 having the insulating layer 123. Since the second electrode 112 is wound on the insulating layer 123 of the first conductor wire 1211, the second electrode 112 is not electrically connected to the first electrode 111. The first electrode 111 and the second electrode 112 may perform independent electrical stimulation functions, respectively, greatly enriching the diversity of treatment.

At present, the spinal percutaneous implanted lead on the market has a minimum diameter of 1.27 mm. The percutaneous implanted lead needs a relatively thick 14G puncture needle (G is the specification of Birmingham Wire Gauge (BWG); the bigger "G", the thinner the outer diameter of needle tube). Such thick needles are difficult to implant in peripheral nerves. Thus, the clinical need for peripheral nerves is to design the percutaneous implanted lead 10 to be 0.70 mm in diameter, which is a size range that is challenging and difficult to design and manufacture. Within this diameter range of the percutaneous implanted lead 10, an 18G needle may be designed based on the syringe structure to effectively and smoothly deliver or implant the percutaneous implanted lead 10 around the painful nerve desired by the physician. Therefore, it is also a great challenge to design the two-stranded spiral conductor wire 121 with a limited cross-sectional area and resisting fatigue distortion and bending, and insulation between the two-stranded conductor wire 121. The conductor wire 121 provided in this example is selected from stainless steel wire or cables. MP35N non-magnetic nickel-cobalt-chromium-molybdenum alloy wire or cables is convoluted to form an elastic elongated spiral conductor wire 121. The cable is formed by twisting multiple strands of stainless steel wire and may have better fatigue resistance. The elasticity and flexibility of the helical conductor wire 121 may overcome muscle tension and compression in vivo. The material design of the insulating layer 123 of the two-strand conductor wire 121 includes a polymer material PU, PTFE or ETFE to be extruded into a tube type and applied to the outer layer of the metal wire 122, or the polymer material PU, PTFE or ETFE sprayed onto the metal wire 122 by the coating process to form the insulating layer 123, so as to ensure the insulation between the first conductor wire 1211 and the second conductor wire 1212. Both the extrusion and spraying processes need to ensure that the insulation wall is uniform, so as to ensure the insulation performance between the first conductor wire 1211 and the second conductor wire 1212 during the implantation treatment. The diameter of the metal wire 122 is preferably 0.1 mm-0.15 mm. The outer diameters of the first electrode 111 and the second electrode 112 are preferably 0.55 mm-0.70 mm. In the peripheral nerve stimulation system provided in this example, although the bipolar percutaneous implanted lead 10 is used, since the first electrode 111 and the second electrode 112 are coaxially spaced with equal diameter, the first conductor wire 1211 and the second conductor wire 1212 are insulated and then wound into an open insulating spiral structure. The percutaneous implanted lead 10 with the first electrode 111 and the second electrode 112 may be implanted into the body using an implantation tool (puncture needle) with the same diameter size of the existing similar products, which is also less invasive but may provide a more effective treatment for nerve regulation.

With continued reference to Fig. 6, the distal end of the first conductor wire 1211 and the distal end of the second conductor wire 1212 are both exposed metal wires 122. The metal wire 122 at the distal end of the second conductor wire 1212 is connected to the second electrode 112 by welding or crimping. The metal wire 122 at the distal end of the first conductor wire 1211 is connected to the first electrode 111 by welding or crimping.

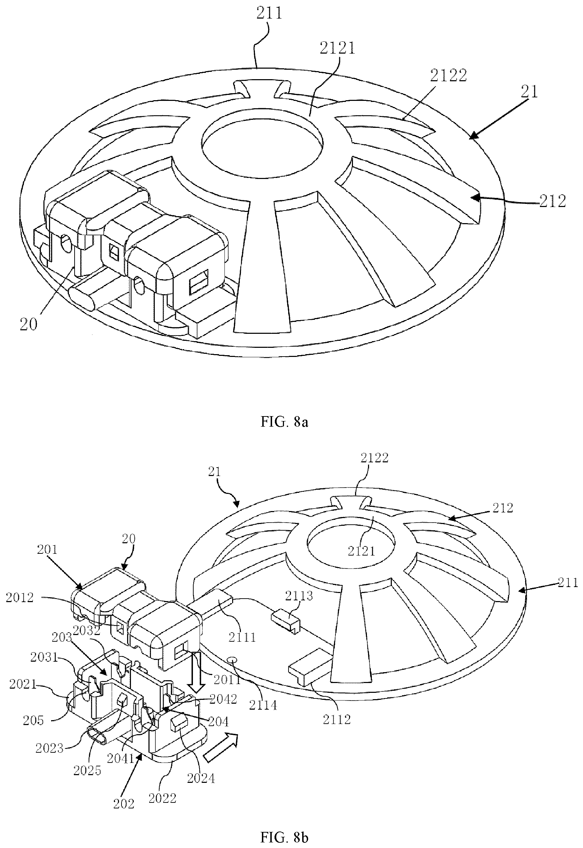

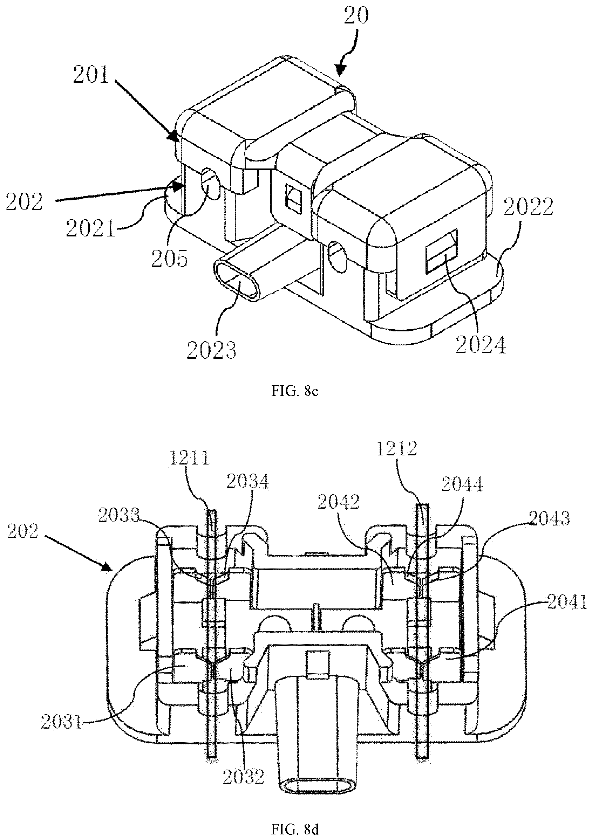

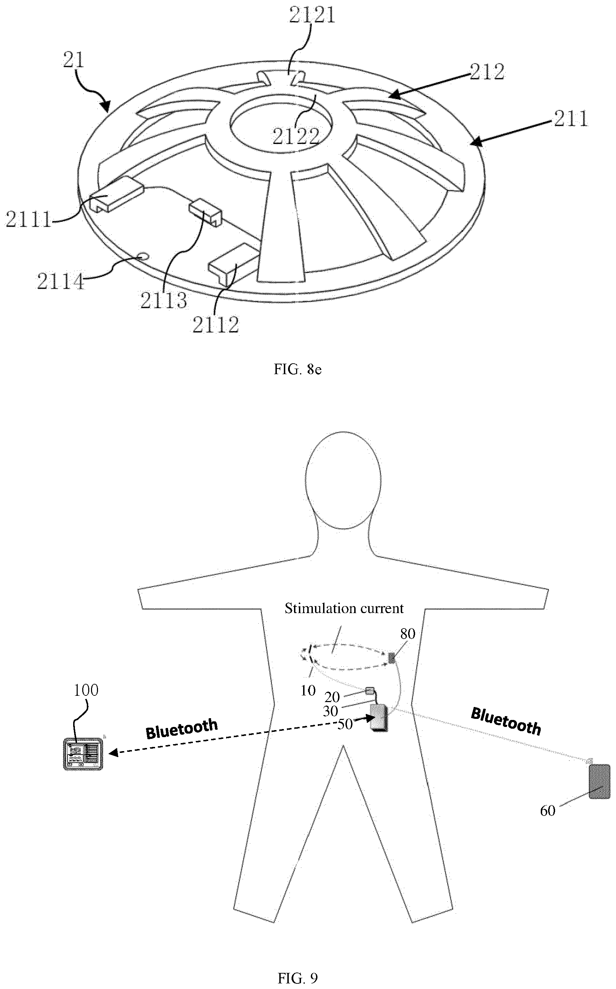

Referring to Figs. 8a-8e, in a specific example, the adapter 20 is mounted on the mounting seat 21 including a main body portion 212 and a base 211. The main body portion 212 is disposed on the base 211. The main body portion 212 is a hollowed-out structure. A skin-friendly adhesive is arranged on the bottom surface of the base 211. The mounting seat 21 may be adhered to the periphery of a wound of a patient by means of the skin-friendly adhesive. The hollowed-out structure of the main body portion 212 may ensure the ventilation effect near to the wound and reduce the infection probability of the wound. Further, a release paper having a skin-friendly adhesive is provided on the bottom surface of the base 211. When the release paper is torn off during use, the mounting seat 21 can be pasted on the skin around the wound 2 as shown in Fig. 1. The outer contours of the top 2121 of the base 211 and the main body portion 212 are both circular, hollowed out in the middle and coaxially provided. The outer diameter of the top 2121 of the main body portion 212 is less than the outer diameter of the base 211. A plurality of arch strips 2122 are connected between the top 2121 and the base 211. The number of the arch strips 2122 is preferably 5-8. The adapter 20 is mounted on the base 211 between two adjacent arch strips 2122, and the rest of the arch strips 2122 are equally spaced in the circumferential direction.

With continued reference to Fig. 8b, in a specific embodiment, a first guide rail 2111, a second guide rail 2112 and a first snap 2114 are disposed on the base 211. A first side wing 2021 and a second side wing 2022 are correspondingly provided on both sides of the bottom of the adapter 20. The first side wing 2021 and the second side wing 2022 are respectively inserted into the first guide rail 2111 and the second guide rail 2112, and are fixed via the first snap 2114. Further, a limiting piece 2113 is disposed on the base 211 for blocking the adapter 20 and preventing the adapter 20 from sliding out of the base 211 after being inserted into the first guide rail 2111 and the second guide rail 2112. The adapter 20 includes a first housing 201 and a second housing 202 which are matingly and detachably connected. In particular, the first housing 201 and the second housing 202 may be snap-connected by providing mating second protrusions 2025 and second slots 2012 on the end sides of the first housing 201 and the second housing 202. A first channel 203 and a second channel 204 are disposed in the second housing 202. A first flexure strip 2031 and a second flexure strip 2032 are oppositely provided in the first channel 203. A third flexure strip 2041 and a fourth flexure strip 2042 are oppositely provided in the second channel 204. The first flexure strip 2031, the second flexure strip 2032, the third flexure strip 2041 and the fourth flexure strip 2042 are all fixed on the second housing 202. In a particular embodiment, the first flexure strip 2031 has a first blade 2033 with an oblique opening. The second flexure strip 2032 has a second blade 2034 with an oblique opening. The third flexure strip 2041 has a third blade 2043 with two oblique openings. The fourth flexure strip 2042 has a fourth blade 2044 with an oblique opening. Yhe first blade 2033 and the second blade 2034 form a V-shaped concave angle with respect to each other. The third blade 2043 and the fourth blade 2044 form a V-shaped concave angle with respect to each other. A through hole 205 is provided on the second housing 202 corresponding to the first channel 203 and the second channel 204. The first conductor wire 1211 passes into the first channel 203 and is placed at the V-shaped concave angle formed by the first blade 2033 and the second blade 2034. The second conductor wire 1212 passes into the second channel 204 and is placed at the V-shaped concave angle formed by the third blade 2043 and the fourth blade 2044. The number of the first blade 2033, the second blade 2034, the third blade 2043 and the fourth blade 2044 is preferably two, which may be designed by a person skilled in the art according to practical requirements. The second housing 202 is provided with an interface 2023 through which the first cable 30 is connected to the first channel 203 and the second channel 204, respectively. During the installation, when the first housing 201 is opened, the first cable 30 is inserted from the interface 2023, welded to the first channel 203 and the second channel 204, respectively, and then fixed at the interface 2023. It may be fixed by spot gluing or other means, and the present invention is not particularly limited thereto. Then, the first housing 201 is snap-fit on the second housing 202, so that the snap on the first housing 201 and the second housing 202 are in an open state. After the percutaneous implanted lead 10 passes out of the wound 2 of the patient, the first conductor wire 1211 at the proximal lead end 13 is placed in the V-shaped concave angle formed by the first blade 2033 and the second blade 2034. The second conductor wire 1212 is placed in the V-shaped concave angle formed by the third blade 2043 and the fourth blade 2044, and snap-fit at the snap on both sides of the first housing 201 and the second housing 202. The snap is a matching structure of the first protrusion 2024 and the first slot 2011. During the snap-fit, the snaps at both sides are mainly used for pressing the first conductor wire 1211 to the gap between the first blade 2033 and the second blade 2034, and pressing the second conductor wire 1212 to the gap between the third blade 2043 and the fourth blade 2044. The sharp first blade 2033, second blade 2034, third blade 2043 and fourth blade 2044 may just cut through the insulating layer 123 of the first conductor wire 1211 and the second conductor wire 1212 to establish a reliable electrical connection, while the snap-fit on both sides fixes the first housing 201 and the second housing 202. Next, the first side wing 2021 and the second side wing 2022 on the bottom of the adapter 20 slide into the first guide rail 2111 and the second guide rail 2112 on the mounting seat 21. The adapter 20 is limited by the limiting piece 2113, and fixed by the first snap 2114.

With regard to the percutaneous implanted lead 10 with a single electrode structure, only one conductor wire 121 is connected to the adapter 20. The conductor wire 121 at the proximal lead end 13 is placed in either one of the first channel 203 and the second channel 204, and the other channel is empty. The specific use mode thereof is the same as that of the percutaneous implanted lead 10 with a double electrode structure, and the description thereof will not be repeated.

Thus, the adapter 20 mounted on the mounting seat 21 may reliably connect the percutaneous implanted lead 10 coming out of the wound 2 with the external stimulator 50 and connect the signal of the percutaneous implanted lead 10 to the stimulator 50. The whole structure provides the protection of the percutaneous implanted lead 10 from the skin wound while allowing the percutaneous implanted lead 10 to stably and reliably conduct the stimulator signal.

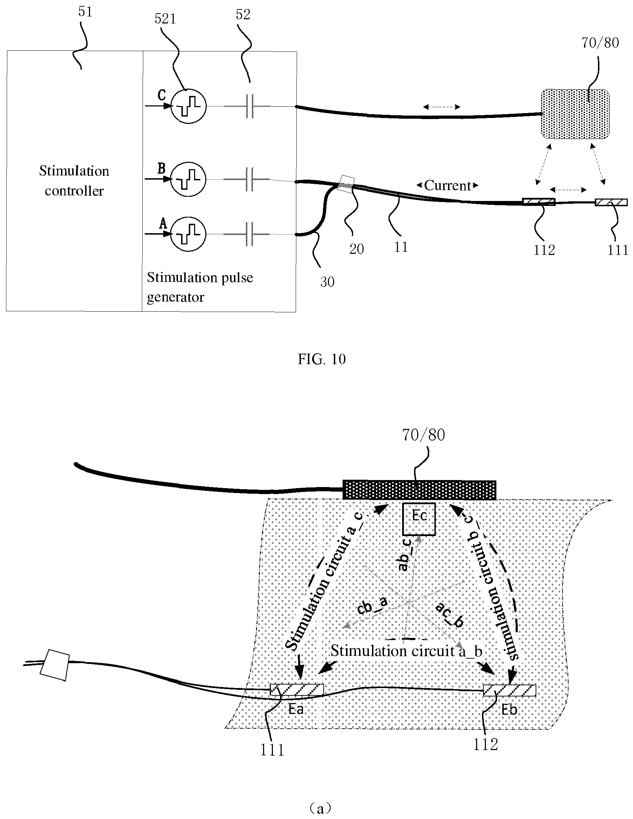

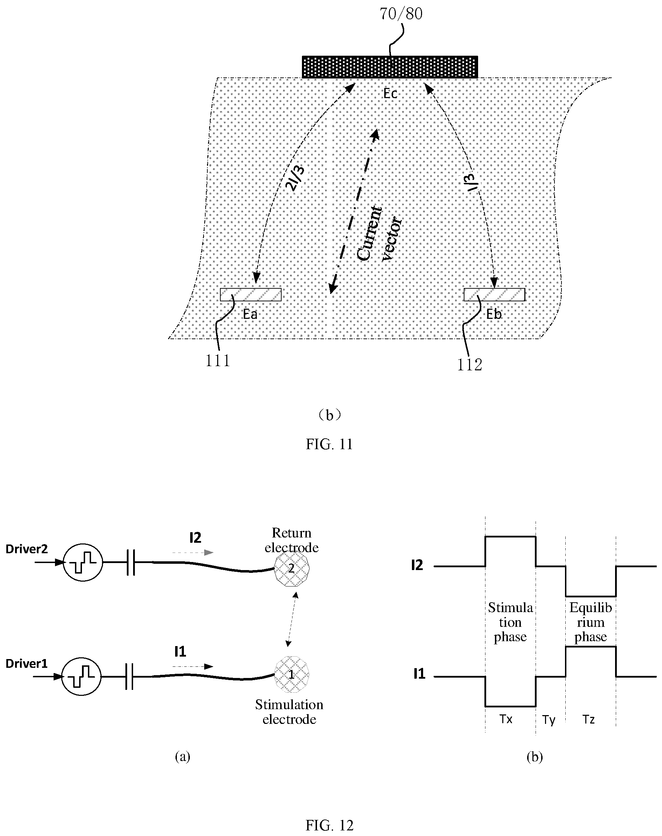

Referring to Fig. 9, the peripheral nerve stimulation system according to the present invention, taking the movable surface electrode 80 as an example, when in use, the percutaneous implanted lead 10 having the first electrode 111 and the second electrode 112 is percutaneously implanted in the vicinity of the target nerve in the body of the patient 3. The adapter 20 is placed on the skin of the patient 3. The stimulator 50 is fixed to the body surface of the patient 3. The movable surface electrode 80 is placed at a place where the patient 3 needs stimulation via the second cable 72. During the treatment, the patient 3 selects the stimulation program and stimulation intensity by the remote controller 60. The stimulation treatment is performed on the target site by the stimulator 50. In addition, the system includes a clinical programmer 100 implemented by the physician using a tablet computer. During the electrode implantation, the physician determines the integrity and function of the electrode by operating the control interface of the clinical programmer 100. In another embodiment, the physician debugs and determines the stimulation scheme (program) by the programming interface of the clinical programmer 100, including waveform, modulation method and safe stimulation intensity range, and solidifies these parameters into the stimulator 50. The patient uses the remote controller 60 to select a certain stimulation program and stimulation intensity by selecting the number form. The Bluetooth communication transmits the control information to the stimulator 50. The stimulator 50 generates a specific current pulse sequence according to the selected program, and outputs it to the target nerve tissue via the external lead, the adapter 20 and one of the percutaneous implanted leads. The stimulation loop is composed of another percutaneous implanted lead or a designated surface electrode. Referring to Fig. 10, the stimulator 50 is a three-channel bi-directional current pulse stimulator including a stimulation controller 51 and a pulse generator 52 including at least one pulse source 521. The stimulation controller 51 controls the output amplitude, current direction, pulse width and/or timing of the pulse source 521. The stimulation controller 51 is a microprocessor or a stimulator-specific control chip. In another example, the three-channel pulse generator 52 is implemented with three independent bi-directional current pulse sources 521 as electrode driver. Each pulse source 521 is capable of individually controlled for generation of negative (N) and positive (P) current pulses. One of the pulse sources 521 drives the surface electrode 70, and the other two drive two implanted first electrodes 111 and second electrodes 112, respectively. In this example, the output function and performance of each channel is indifferent, i.e., either one or a combination of two electrodes may be used as a stimulation electrode (negative pole) or a return electrode (positive pole), with the remaining one or two electrodes making up the stimulation return. Thus, three electrodes may form six different stimulation pathways consisting of a stimulation electrode and a return electrode , as shown in Fig. 11a. Each stimulation pathway may act on different neural tissues, or there are six possible stimulation options under the same electrode implantation conditions. Furthermore, as shown in Fig. 11b, in the case of a combination of two electrodes, by distributing the current intensity between the two electrodes, the overall flow direction of the current may be controlled, reaching the effect of the virtual electrode, thereby providing a new possibility for improving the stimulation effect. When stimulation occurs, the pulse source of the stimulation electrode and the pulse source of the return electrode form a "bridge" push-pull output mode, as shown in Fig. 12a. In the stimulation phase, the current flows from the positive pole to the negative pole, while in the equilibrium phase, the current is reversed, as shown in Fig. 12b.

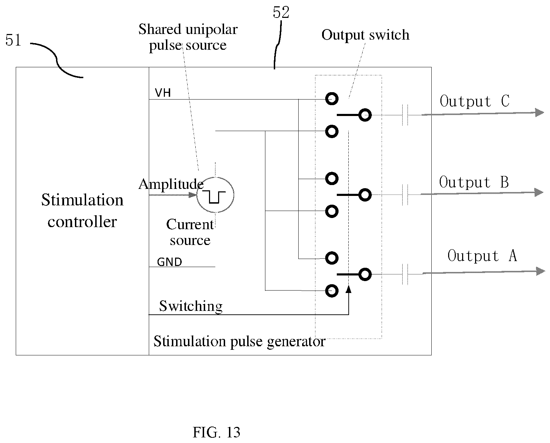

Referring to Fig. 13, in another example, the stimulator 50 includes a single unidirectional current pulse source. In this example, three stimulation channels share an unidirectional current pulse source driver. The stimulation pulse output by the driver is distributed to a designated electrode or electrodes by an output switch, i.e., the stimulation pulse output by the current pulse source is distributed to the surface electrode 70 or 80, the first electrode 111 or/and the second electrode 112 by the output switch. Stimulation and equilibrium phase direction switching of the current pulses is also achieved by the output switch. This design also allows six stimulation loops to be implemented by the electrode combination, as shown in Fig. 11a. However, this design does not allow for a controllable virtual electrode as shown in Fig. 1 lb. This design may achieve the effect of reducing circuit power consumption and reducing circuit components.

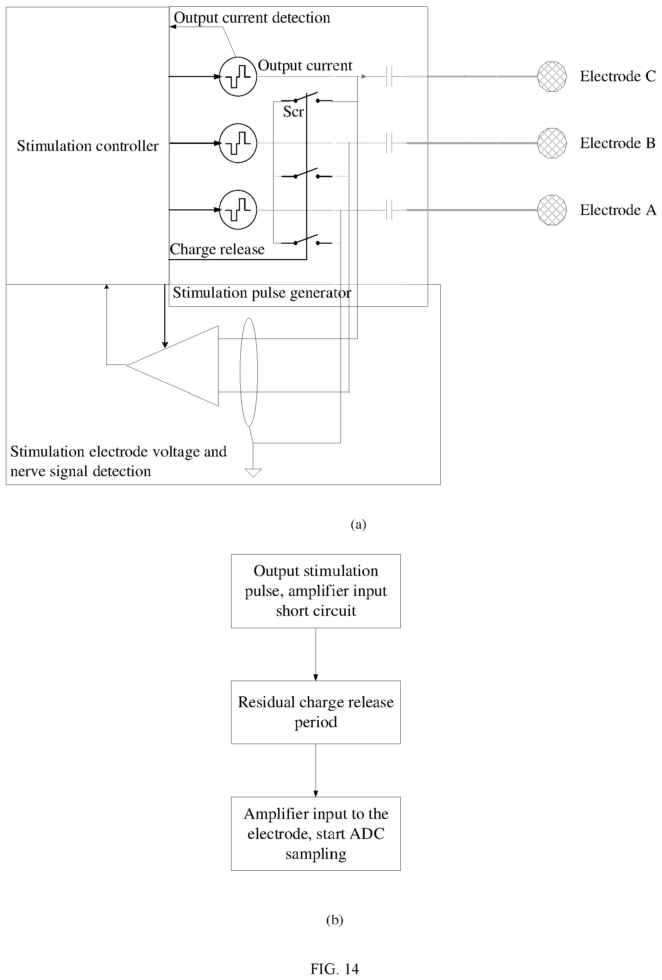

In any of the above embodiments, the stimulator may measure the impedance of the percutaneous electrode by outputting a fixed small current pulse while monitoring the voltage of the percutaneous electrode to determine the integrity of the percutaneous electrode. At the same time, the dual-percutaneous electrode structure also facilitates the detection of nerve response signals to stimulation pulses. In the embodiment shown in Fig. 14a, the nerve signal detection circuit adopts a stimulation electrode as a detection electrode, while using a non-stimulation electrode as a reference electrode or signal ground. The nerve signal detection process is reflected in the embodiment as shown in Fig. 14b. Firstly, the stimulator outputs a current pulse to stimulate the target nerve tissue, and also the nerve signal detection channel is closed so as not to block the amplifier with high gain. After the stimulation pulse is completed, a charge release period is immediately added, and the residual charge left on the output capacitor by the stimulation pulse is released by the short-circuit switch, so as to reduce the interference such as DC drift of the electrode potential. At the end of the charge release period, the neural signal detection channel is conducted to record the response signal of the nerve formed by the stimulation-evoked action potential. All the timing of the signal detection process is set according to the signal conduction characteristics of the target nerve to achieve the best recording effect. The recorded neural response signals may be used for clinical diagnosis and also as feedback of stimulation to adjust the stimulation intensity.

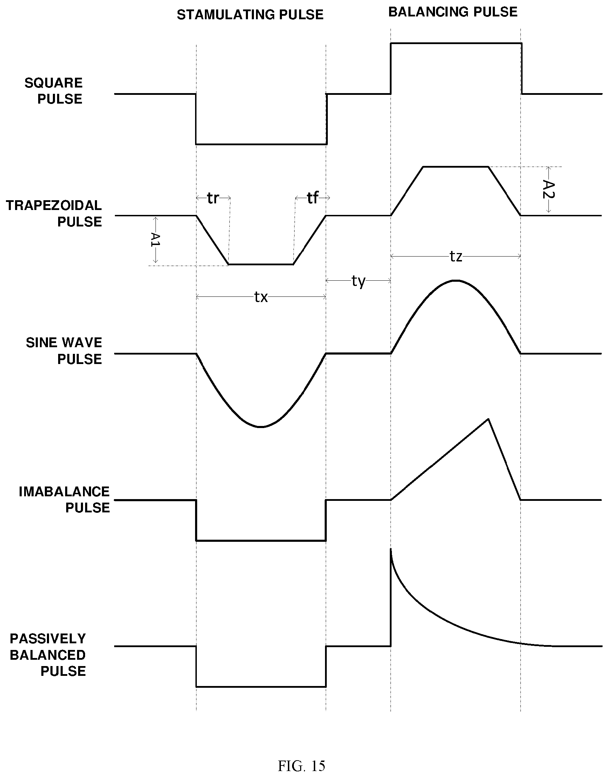

Referring to Fig. 15, the stimulation system provided by the present invention adds four new current stimulation pulse waveform templates, inclduing trapezoidal, sinewave, asymmetric, and passively balanced wave, in addition to the default square wave stimulation pulse. Each waveform block is stimulated by a stimulation phase pulse of amplitude Al and width tx, followed by an equilibrium phase pulse of amplitude A2 and width tz responsible for charge neutralization. An adjustable inter-wave delay ty is set between the two-phase pulses. If the equilibrium phase is set to a current pulse for rapid neutralization, it is an initiative balance stimulation. Regardless of the waveform template used, the safety of stimulation requires that the charge of the stimulation phase and the equilibrium phase be equal, i.e., Q1 = Q2, where Q1 and Q2 are the integrals of the current pulse waveform over the pulse width, i.e., $Q = \int_0^{T} idt$. At the same time, the maximum stimulation intensity of monophasic pulse is also restricted by Shannon Criteria. That is, K=Log(D*Q) ≤1.85, where D is the current density of the monophasic pulse in milliamps per square centimeter, which is related to the electrode surface area. Q is the amount of monophasic stimulation charge, which is related to the current amplitude and pulse width. Recent studies have shown that for peripheral nerve stimulation, the K value may exceed 1.85, but this limit may be adjusted by the software of the stimulator. The equilibrium phase may also be stimulated in the form of a short form of a short circuit discharge, i.e., passive equilibrium. Passive equilibrium stimulation may save the stimulation current of equilibrium phase, but the required discharge time is long, which is not suitable for high frequency stimulation. If the stimulation phase and the equilibrium phase are in the same pulse form, it is symmetric stimulation, whereas if different pulse forms are used, it is asymmetric stimulation. According to the asymmetric stimulation, the charge of two stimulation phases may be unbalanced, but the sum of the charge of stimulation pulse and the charge of equalizing pulse shall be equalized within a certain time interval. The default value of each parameter of stimulation pulse waveform is adjusted to the optimal default value through clinical trial, but the physician may further adjust them by the clinical programmer according to the patient's response effect to stimulation.

Both the trapezoidal and sinusoidal waves have slow rising and falling edges to reduce the high frequency components of the stimulation pulse. Reported clinical studies have shown that the stimulation or electric field changes with high frequency components tend to activate nerves close to the electrodes, while the low-frequency stimulation allows stimulation-induced electric field changes to propagate further, which is thus effective for nerves at a distance from the electrodes. The position of the electrode after implantation is fixed, so that the relative position of the electrode and each nerve tissue is also fixed. The trapezoidal and sinusoidal waves offer us the possibility of targeted and selective stimulation of the target nerve after electrode implantation.

In addition to providing a variety of selection of stimulation waveforms, the stimulator also provides a variety of stimulation modes, i.e., dynamically modulating the amplitude or frequency of a stimulation pulse sequence in different ways, and outputting the modulated pulse sequence (referred to as a stimulation module) continuous or burst at a specified time, a specified manner, and a specified intensity range by the scheduling of the stimulation program. Examples of the stimulation mode provided by the present invention include the followings.

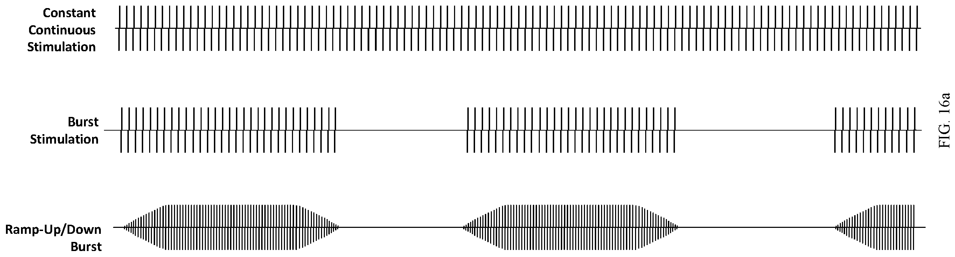

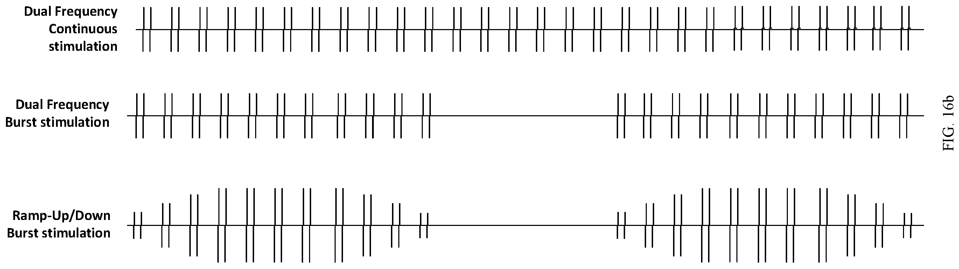

1. Constant frequency and amplitude stimulation mode: the stimulator outputs continuous stimulation pulses to designated electrodes according to the selected stimulation pulse waveform, frequency and amplitude, as shown in Fig. 16a. The stimulation program selects continuous output or burst output modes and specifies the pulse frequency and the upper and lower limits of adjustable amplitude. The patient may adjust the stimulation intensity, i.e., the amplitude between the upper and lower limits of the stimulation pulse, according to the perceived effect. The patient with burst output may also choose Ramp-up and Ramp-down to allow the stimulation intensity to increase or decrease smoothly and increase comfort. In addition, the stimulation pattern includes a dual frequency stimulation sequence, as shown in Fig. 16b. The dual-frequency stimulation was added with a slow interference frequency to the main frequency, which may be used in some special stimulation cases.

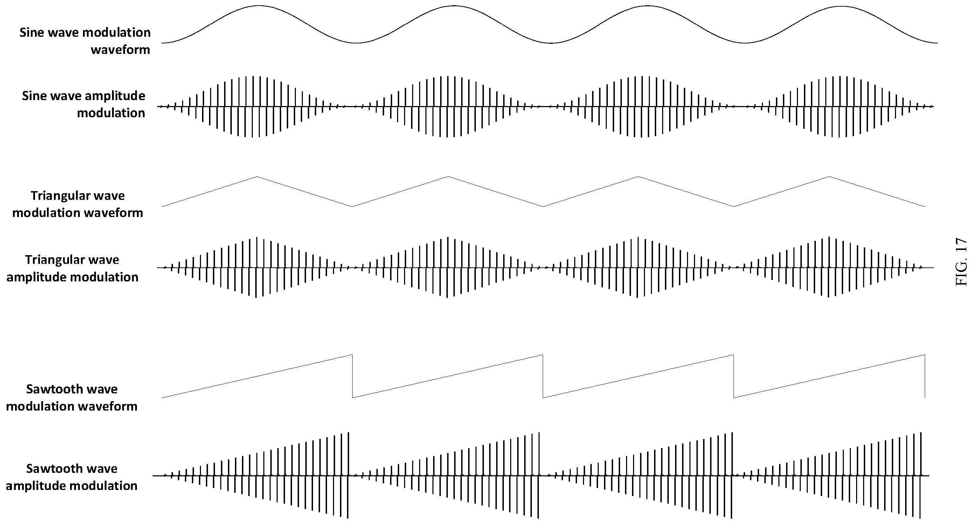

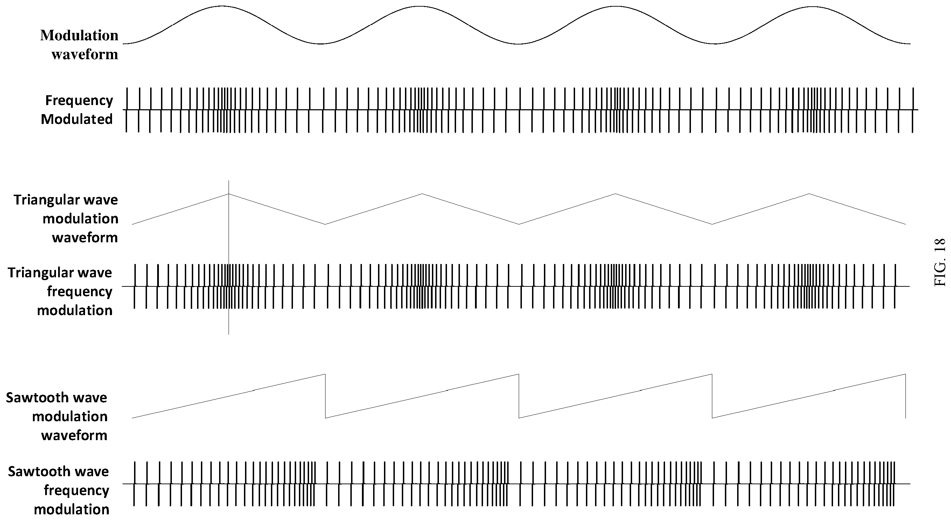

2. Simulated (continuous) modulation stimulation mode: the stimulator continuously modulates the amplitude or frequency of the selected stimulation pulse waveform in different simulation ways, as shown in Fig. 17 and Fig. 18, respectively. The simulation modulation manners include sine wave modulation, triangular wave modulation, and sawtooth wave modulation. This pattern adds a modulation frequency, called interference frequency, to the basic stimulation elements. The frequency of interference may have some stimulation effect on specific symptoms. However, the purpose of this mode of stimulation is to reduce or even eliminate the rapid loss of therapeutic effect due to habituation and fatigue caused by extracellular stimulation. The control parameters of the simulation modulation mode include the modulation period, the average amplitude value or average frequency value, and the modulation depth percentage, from which the maximum and minimum values of amplitude or frequency may be determined.

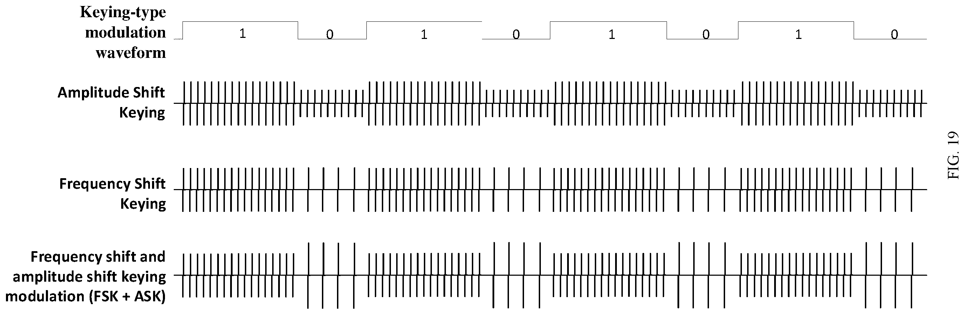

3. Keying-type shift modulation stimulation mode: the stimulator performs two-level hopping switching of amplitude or frequency or both of amplitude shift keying and frequency shift keying at specified intervals on the selected stimulation pulse waveform, as shown in Fig. 19. The study on the principle of percutaneous electrical stimulation analgesia shows that the analgesia effect of interactive stimulation with low-frequency high-current and high-frequency low-current is better than that of single-frequency or high-frequency and low-frequency stimulation at different stimulation points. The adjustment parameters of the keying-shift stimulation include high and low amplitude, high and low frequency, modulation frequency, and modulation duty cycle, i.e., the proportion of high value time in the modulation period.

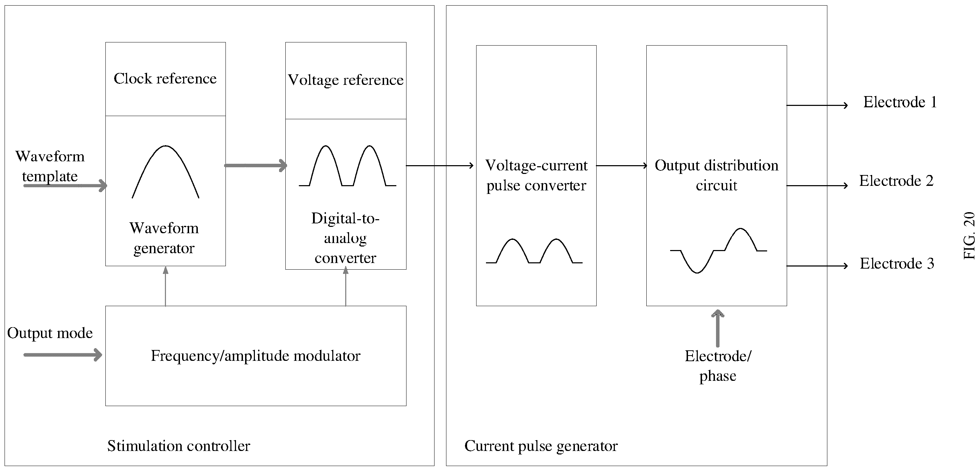

Referring to Fig. 20, in a stimulator circuit example, the stimulation waveform and modulation stimulation mode functions of the stimulator are implemented primarily by a stimulation controller. The stimulation controller includes a waveform generator, a clock reference, a voltage reference, a digital-to-analog converter, and an amplitude and frequency modulator.

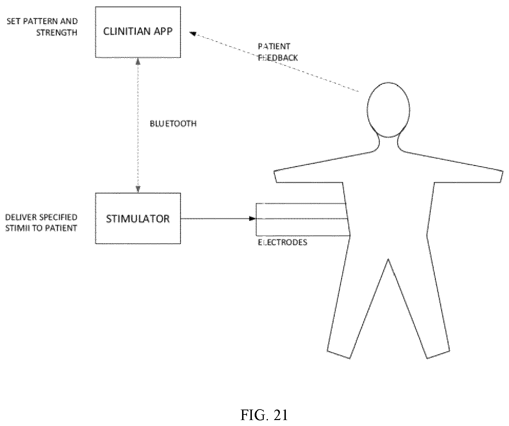

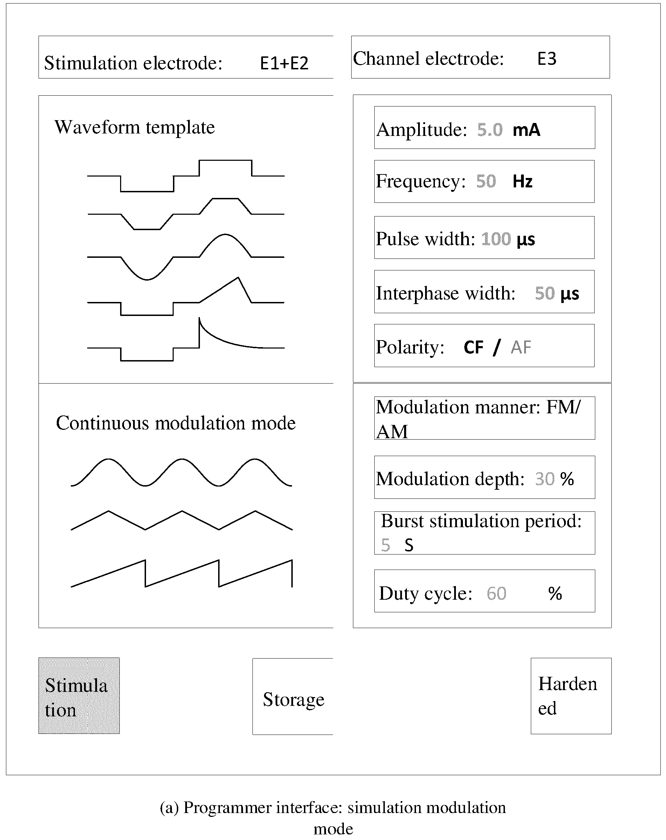

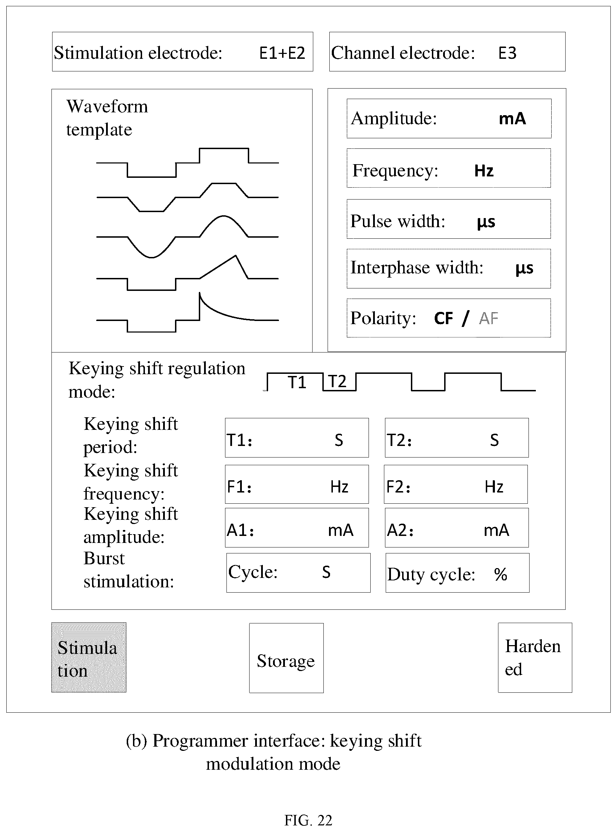

Referring to Fig. 21, the nerve stimulation system provides a patient specific stimulation plan, or "stimulation prescription". Each patient's stimulator has a specific stimulation plan, including an appropriate stimulation waveform, a stimulation program and an effective range of stimulation intensity, etc. The stimulation plan is implemented through the patient fitting process. The patient fitting is performed in a hospital or clinic and the required setup is shown in Fig. 21. Through the physician's programming interface (Clinician App) of clinical programmer, the physician selects the parameters such as stimulation electrode, stimulation waveform, stimulation mode and stimulation intensity, so as to directly control the stimulator to perform stimulation. According to the patient's feedback, the physician evaluates the selected stimulation parameters, so as to determine the stimulation electrode or electrode combination, stimulation mode, stimulation program and corresponding range of effective stimulation intensity applicable to this patient. As a result of patient fitting, a stimulation prescription suitable for the patient is obtained, sintered in the form of a table in the hardened memory of the stimulator. The patient selects the corresponding table directory number of the stimulator in the memory via the remote controller, including the stimulation program and stimulation intensity. Fig. 22 is an example of a programming interface for a physician's clinical programmer.

In summary, the invention has at least the following advantages. 1. The unipolar or bipolar electrode is implanted by the implantation tool with smaller diameter than that of the introducer of similar products. The diameter of the percutaneous part of lead body may be reduced to the diameter of metal wire plus the thickness of wrapped insulating layer, so as to reduce the trauma and provide more effective treatment for nerve regulation. 2. The dual-percutaneous electrode combined with a novel surface electrode is provided to arbitrarily selected stimulation loop, which increases the stimulation range and improves the treatment effectiveness; 3. It provides more effective neuromodulation by the novel stimulation waveforms and the pulse modulation manner. 4. It provided a novel wound protection structure, while connecting a tiny electrode to the stimulator by the cable, so that it may reduce the chance of accidental wound injury and infection during the treatment of the patient. 5. The movable surface electrodes enable the increased stimulation range and more effective neuromodulation.

Although the present invention has been described with reference to the preferred examples, it is not intended to limit the present invention. Any person skilled in the art may modify and improve it somewhat without deviating from the scope of the present invention as defined in the claims.

**Claims**

1. A peripheral nerve stimulation system, comprises a percutaneous implanted lead (10), an adapter (20), a first cable (30), a stimulator (50), and a surface electrode;

    the percutaneous implanted lead (10) can be inserted into the body of a patient through skin, and the percutaneous implanted lead (10) comprises a distal lead head (11) and a lead body (12) from a distal end to a proximal end, the distal lead head (11) comprising at least one electrode, the lead body (12) being formed by spirally winding at least one conductor wire (121), and the conductor wire (121) comprising a metal wire (122) and an insulating layer (123), which is arranged outside the metal wire (122) in a wrapping manner, such that the lead body (12) is of an open insulating spiral structure;
    the adapter (20) is electrically connected to the proximal end of the percutaneous implanted lead (10); the adapter (20) is electrically connected to the stimulator (50) by means of the first cable (30); the stimulator (50) is used for sending an electrical stimulation pulse; and
    the surface electrode is electrically connected to the stimulator (50), and forms a stimulation loop together with the percutaneous implanted lead (10) through a stimulated human tissue;
    **characterized by** the peripheral nerve stimulation system further comprising a remote controller (60) for a patient, the remote controller (60) being connected to the stimulator (50) by Bluetooth communication; and the remote controller (60) stores stimulation program ordinals and stimulation intensity ordinals corresponding to the contents of a program table and an intensity table in a hardened memory of the stimulator (50), respectively.

2. The peripheral nerve stimulation system according to claim 1, **characterized in that** the distal lead head (11) comprises a first electrode (111) and a fixation hook (113); the at least one conductor wire (121) having one conductor wire (121); the spiral turns constituting the lead body (12) are sequentially arranged at a predetermined pitch in an axial direction; the first electrode (111) is formed in a distal direction under an exposed state by spirally winding the metal wire (122) constituting the conductor wire (121); and the metal wire (121) at the distal end of the first electrode (111) continues to extend distally and is bent to form the fixation hook (113); the pitch of the first electrode (111) between two adjacent turns within 2-3 turns of the distal end thereof is greater than the pitch elsewhere.

3. The peripheral nerve stimulation system according to claim 1, **characterized in that** the distal lead head (11) comprises a first electrode (111) and a fixation hook (113); wherein the first electrode (111) is a tubular metal electrode; the at least one conductor wire (121) having one conductor wire (121); the spiral turns constituting the lead body (12) are sequentially arranged at a predetermined pitch along the axial direction; the metal wire (122) constituting the conductor wire (121) extends distally through an electrode tube of the metal electrode under an exposed state and continues to extend and bend to form the fixation hook (113); and the metal wire (122) is electrically connected with the metal electrode.

4. The peripheral nerve stimulation system according to claim 1, **characterized in that** the distal lead head (11) -comprises a first electrode (111) and a fixation hook (113), wherein the first electrode (111) is a tubular metal electrode; the at least one conductor wire (121) having one conductor wire (121); the spiral turns constituting the lead body (12) are sequentially arranged at a predetermined pitch along the axial direction; a distal end of the metal wire (122) constituting the conductor wire (121) extends and then extends into a proximal end of an electrode tube of the first electrode (111) and is electrically connected to the first electrode (111); the fixation hook (113) is made of the metal wire (122); and the proximal end of the fixation hook (113) extends into a distal end of the electrode tube of the first electrode (111) and is connected to the first electrode (111).

5. The peripheral nerve stimulation system according to claim 1, **characterized in that** the distal lead head (11) comprises a second electrode (112), a first electrode (111) and a fixation hook (113) in sequence from the proximal end to the distal end, wherein the first electrode (111) and the second electrode (112) are arranged along a same axial interval and insulated; the at least one conductor wire (121) having a first conductor wire (1211) and a second conductor wire (1212); the spiral turns constituting the lead body (12) are sequentially arranged at a predetermined pitch along the axial direction; the first electrode (111) is connected to the adapter (20) via the first conductor wire (1211); the second electrode (112) is connected to the adapter (20) via the second conductor wire (1212); any one electrode or a combination of any two electrodes of the first electrode (111), the second electrode (112) and the surface electrode being used as a stimulation electrode or a return electrode , and constitutes a stimulation loop together with the remaining one or two electrodes through a stimulated human tissue.

6. The peripheral nerve stimulation system according to claim 5, **characterized in that** the first electrode (111) and the second electrode (112) are both tubular metal electrodes; the first conductor wire (1211) is insulated to pass through the electrode tube of the second electrode (112); the metal wire (122) constituting the first conductor wire (1211) extends distally through the electrode tube of the first electrode (111) and then continues to extend distally and bend to form the fixation hook (113), the metal wire (122) of the first conductor wire (1211) being electrically connected to the first electrode (111); the exposed metal wire (122) of the second conductor wire (1212) at the distal end extends into the electrode tube of the second electrode (112) and is electrically connected to the second electrode (112); or, the metal wire (122) constituting the second conductor wire (1212) extends distally under an exposed state and is spirally wound to form the second electrode (112); the distal end of the first conductor wire (1211) is insulated to pass through a cavity spirally formed by the second electrode (112); the metal wire (122) constituting the first conductor wire (1211) extends distally under an exposed state and is spirally wound to form the first electrode (111); and the metal wire (122) of the first electrode (111) at the distal end continues to extend distally and is bent to form the fixation hook (113).

7. The peripheral nerve stimulation system according to claim 6, **characterized in that** the first conductor wire (1211) between the first electrode (111) and the second electrode (112) is provided with an insulating buffer sleeve (114); the bending angle of the fixation hook is 20-60 degrees; the metal wire (122) has a diameter of 0.1 mm-0.15 mm, and the outer diameter of the first electrode (111) or/and the second electrode (112) is 0.55 mm-0.70 mm; the exposed metal wire (122) is welded or crimped to the first electrode (111) or the second electrode (112).

8. The peripheral nerve stimulation system according to claim 1, **characterized in that** the metal wire (122) is 316L stainless steel wire or MP35N nonmagnetic nickel-cobalt-chromium-molybdenum alloy wire; the insulating material used for the insulating layer (123) is polyurethane, teflon or ethylene-tetrafluoroethylene copolymer; and the insulating material is extruded to a tubular form and then applied to the periphery of the metal wire (122), or the insulating material is sprayed onto the metal wire (122) by using a coating process.

9. The peripheral nerve stimulation system according to claim 1, **characterized by** further comprising a mounting seat (21); the adapter (20) is mounted on the mounting seat (21); the mounting seat (21) comprises a main body portion (212) and a base (211); the main body portion (212) is disposed on the base (211); the main body portion (212) has an arched hollowed-out structure; the bottom surface of the base (211) is provided with a skin-friendly adhesive; and the mounting seat (21) is attachable to a patient's wound periphery by the skin-friendly adhesive; a first guide rail (2111), a second guide rail (2112) and a first snap (2114) are disposed on the base (211); a first side wing (2021) and a second side wing (2022) are respectively disposed on both sides of the bottom of the adapter (20); and the first side wing (2021) is inserted into the first guide rail (2111), the second side wing (2022) is inserted into the second guide rail (2112), and the base (211) and the adapter (20) are fixed by the first snap (2114).

10. The peripheral nerve stimulation system according to claim 9, **characterized in that** the base (211) and the top (2121) of the main body portion (212) are both annular and coaxially disposed; the outer diameter of the top (2121) of the main body portion (212) is less than the outer diameter of the base (211); and a plurality of arch strips (2122) are connected between the top (2121) and the base (211); the number of arch strips (2122) is 5-8; the adapter (20) is disposed on the base (211) between two of the adjacent arch strips (2122); the remaining plurality of arch strips (2122) are equally spaced in a circumferential direction.

11. The peripheral nerve stimulation system according to claim 5, **characterized in that** the adapter (20) comprises a first housing (201) and a second housing (202) matingly and detachably connected, a first channel (203) and a second channel (204) being disposed in the second housing (202); a first flexure strip (2031) and a second flexure strip (2032) are oppositely disposed in the first channel (203); a third flexure strip (2041) and a fourth flexure strip (2042) are oppositely provided in the second channel (204); the first flexure strip (2031) has a first blade (2033) with an oblique opening, the second flexure strip (2032) having a second blade (2034) with an oblique opening, the third flexure strip (2041) having a third blade (2043) with an oblique opening, and the fourth flexure strip (2042) having a fourth blade (2044) with an oblique opening; the first blade (2033) and the second blade (2034) form a V-shaped concave angle with respect to each other, the third blade (2043) and the fourth blade (2044) forming a V-shaped concave angle with respect to each other; the first conductor wire (1211) passes into the first channel (203) and is placed at the V-shaped concave angle formed by the first blade (2033) and the second blade (2034); the second conductor wire (1212) passes into the second channel (204) and is placed at the V-shaped concave angle formed by the third blade (2043) and the fourth blade (2044); when the first housing (201) is pressed against the second housing (202), the first blade (2033) and the second blade (2034) cooperate to cut the insulating layer (123) of the first conductor wire (1211), and the third blade (2043) and the fourth blade (2044) cooperate to cut the insulating layer (123) of the second conductor wire (1212) to electrically connect the first conductor wire (1211) and the second conductor wire (1212) with the adapter (20); and an interface (2023) is disposed on the second housing (202), and the first cable (30) is connected to the first channel (203) and the second channel (204), respectively, via the interface (2023).

12. The peripheral nerve stimulation system according to claim 1, **characterized in that** the surface electrode is a fixed surface electrode (70), the fixed surface electrode (70) being disposed at the bottom of the stimulator (50), and the fixed surface electrode (70) being in direct electrical connection with the stimulator (50); or the surface electrode is a movable surface electrode (80) which is electrically connected to the stimulator (50) by the second cable (72); and the movable surface electrode (80) can be placed at a desired part of the human body.

13. The peripheral nerve stimulation system according to claim 1, **characterized in that** the remote controller (60) comprises a hand grip (64), a control key (61), a display screen (62), and a hanging hole (63), wherein the control key (61) is disposed at an end portion of the hand grip (64), the hanging hole (63) is disposed at a tail portion of the hand grip (64), and the display screen (62) is disposed at a top surface of the hand grip (64); or, the remote controller (60) comprises a hand grip (64), a control key (61), a display screen (62) and a charging interface (65), wherein the control key (61) and the display screen (62) are disposed on a top surface of the hand grip (64), and the charging interface (65) is disposed on a side surface of the hand grip (64).

14. The peripheral nerve stimulation system according to claim 1, **characterized by** comprising a clinical programmer (100) implemented with a tablet computer, wherein a clinician sets or adjusts stimulation parameters and stimulation programs of the stimulator and diagnoses conditions of the percutaneous implanted lead (10) for the patient during the implantation or treatment stage by a programming interface of the clinical programmer.

**Patentansprüche**

1. System zur peripheren Nervenstimulation, umfassend eine perkutane implantierte Leitung (10), einen Adapter (20), ein erstes Kabel (30), einen Stimulator (50) und eine Oberflächenelektrode;

   wobei die perkutane implantierte Leitung (10) durch Haut in den Körper eines Patienten eingeführt werden kann und die perkutane implantierte Leitung (10) einen distalen Leitungskopf (11) und einen Leitungskörper (12) von einem distalen Ende zu einem proximalen Ende umfasst, wobei der distale Leitungskopf (11) mindestens eine Elektrode umfasst, wobei der Leitungskörper (12) durch spiralförmiges Wickeln mindestens eines Leiterdrahts (121) gebildet wird und der Leiterdraht (121) einen Metalldraht (122) und eine Isolierschicht (123) umfasst, die außerhalb des Metalldrahts (122) auf umhüllende Weise eingerichtet ist, so dass der Leitungskörper (12) eine offene isolierende Spiralstruktur ist; der Adapter (20) elektrisch mit dem proximalen Ende der perkutanen implantierten Leitung (10) verbunden ist; wobei der Adapter (20) mithilfe des ersten Kabels (30) elektrisch mit dem Stimulator (50) verbunden ist; wobei der Stimulator (50) zum Senden eines elektrischen Stimulationsimpulses verwendet wird; und die Oberflächenelektrode elektrisch mit dem Stimulator (50) verbunden ist und zusammen mit der perkutanen implantierten Leitung (10) eine Stimulationsschleife durch ein stimuliertes menschliches Gewebe bildet; **dadurch gekennzeichnet, dass** das System zur peripheren Nervenstimulation ferner eine Fernbedienung (60) für einen Patienten umfasst, wobei die Fernbedienung (60) durch Bluetooth-Kommunikation mit dem Stimulator (50) verbunden ist und die Fernbedienung (60) Stimulationsprogrammordinale und Stimulationsintensitätsordinale umfasst, die dem Inhalt einer Programmtabelle bzw. einer Intensitätstabelle in einem geschützten Speicher des Stimulators (50) entsprechen.

2. System zur peripheren Nervenstimulation nach Anspruch 1, **dadurch gekennzeichnet, dass** der distale Leitungskopf (11) eine erste Elektrode (111) und einen Fixierungshaken (113) umfasst; der mindestens eine Leiterdraht (121) einen Leiterdraht (121) aufweist; die Spiralwindungen, die den Leitungskörper (12) bilden, sequenziell in einem vorbestimmten Abstand in einer Axialrichtung eingerichtet sind; die erste Elektrode (111) in einer distalen Richtung unter einem freigelegten Zustand durch spiralförmiges Wickeln des Metalldrahts (122), der den Leiterdraht (121) bildet, gebildet wird und der Metalldraht (121) am distalen Ende der ersten Elektrode (111) fortfährt, sich distal zu erstrecken, und gebogen ist, um den Fixierungshaken (113) zu bilden; wobei der Abstand der ersten Elektrode (111) zwischen zwei angrenzenden Windungen innerhalb von 2-3 Windungen des distalen Endes davon größer als der Abstand an anderer Stelle ist.

3. System zur peripheren Nervenstimulation nach Anspruch 1, **dadurch gekennzeichnet, dass** der distale Leitungskopf (11) eine erste Elektrode (111) und einen Fixierungshaken (113) umfasst; wobei die erste Elektrode (111) eine röhrenförmige Metallelektrode ist; der mindestens eine Leiterdraht (121) einen Leiterdraht (121) aufweist; die Spiralwindungen, die den Leitungskörper (12) bilden, sequenziell in einem vorbestimmten Abstand entlang der Axialrichtung eingerichtet sind; sich der Metalldraht (122), der den Leiterdraht (121) bildet, distal durch eine Elektrodenröhre der Metallelektrode unter einem freigelegten Zustand erstreckt und fortfährt, sich zu erstrecken und zu biegen, um den Fixierungshaken (113) zu bilden; und der Metalldraht (122) elektrisch mit der Metallelektrode verbunden ist.

4. System zur peripheren Nervenstimulation nach Anspruch 1, **dadurch gekennzeichnet, dass** der distale Leitungskopf (11) eine erste Elektrode (111) und einen Fixierungshaken (113) umfasst; wobei die erste Elektrode (111) eine röhrenförmige Metallelektrode ist; der mindestens eine Leiterdraht (121) einen Leiterdraht (121) aufweist; die Spiralwindungen, die den Leitungskörper (12) bilden, sequenziell in einem vorbestimmten Abstand entlang der Axialrichtung eingerichtet sind; sich ein distales Ende des Metalldrahts (122), der den Leiterdraht (121) bildet, erstreckt und dann in ein proximales Ende einer Elektrodenröhre der ersten Elektrode (111) erstreckt und elektrisch mit der ersten Elektrode (111) verbunden ist; der Fixierungshaken (113) aus dem Metalldraht (122) hergestellt ist und sich das proximale Ende des Fixierungshakens (113) in ein distales Ende der Elektrodenröhre der ersten Elektrode (111) erstreckt und mit der ersten Elektrode (111) verbunden ist.

5. System zur peripheren Nervenstimulation nach Anspruch 1, **dadurch gekennzeichnet, dass** der distale Leitungskopf (11) eine zweite Elektrode (112), eine erste Elektrode (111) und einen Fixierungshaken (113) der Reihe nach vom proximalen Ende zum distalen Ende umfasst, wobei die erste Elektrode (111) und die zweite Elektrode (112) entlang desselben axialen Intervalls eingerichtet und isoliert sind; der mindestens eine Leiterdraht (121) einen ersten Leiterdraht (1211) und einen zweiten Leiterdraht (1212) aufweist; die Spiralwindungen, die den Leitungskörper (12) bilden, sequenziell in einem vorbestimmten Abstand entlang der Axialrichtung eingerichtet sind; die erste Elektrode (111) mittels des ersten Leiterdrahts (1211) mit dem Adapter (20) verbunden ist; die zweite Elektrode (112) mittels des zweiten Leiterdrahts (1212) mit dem Adapter (20) verbunden ist; eine beliebige eine Elektrode oder eine Kombination von beliebigen zwei Elektroden der ersten Elektrode (111), der zweiten Elektrode (112) und der Oberflächenelektrode als eine Stimulationselektrode oder eine Gegenelektrode verwendet wird und zusammen mit der restlichen einen oder den restlichen zwei Elektroden eine Stimulationsschleife durch ein stimuliertes menschliches Gewebe bildet.

6. System zur peripheren Nervenstimulation nach Anspruch 5, **dadurch gekennzeichnet, dass** die erste Elektrode (111) und die zweite Elektrode (112) beide röhrenförmige Metallelektroden sind; der erste Leiterdraht (1211) isoliert ist, um durch die Elektrodenröhre der zweiten Elektrode (112) hindurchzutreten; sich der Metalldraht (122), der den ersten Leiterdraht (1211) bildet, distal durch die Elektrodenröhre der ersten Elektrode (111) erstreckt und dann fortfährt, sich distal zu erstrecken und zu biegen, um den Fixierungshaken (113) zu bilden, wobei der Metalldraht (122) des ersten Leiterdrahts (1211) elektrisch mit der ersten Elektrode (111) verbunden ist; sich der freigelegte Metalldraht (122) des zweiten Leiterdrahts (1212) am distalen Ende in die Elektrodenröhre der zweiten Elektrode (112) erstreckt und elektrisch mit der zweiten Elektrode (112) verbunden ist oder sich der Metalldraht (122), der den zweiten Leiterdraht (1212) bildet, distal unter einem freigelegten Zustand erstreckt und spiralförmig gewickelt ist, um die zweite Elektrode (112) zu bilden; das distale Ende des ersten Leiterdrahts (1211) isoliert ist, um durch einen Hohlraum hindurchzutreten, der spiralförmig von der zweiten Elektrode (112) gebildet wird; sich der Metalldraht (122), der den ersten Leiterdraht (1211) bildet, unter einem freigelegten Zustand distal erstreckt und spiralförmig gewickelt ist, um die erste Elektrode (111) zu bilden; und der Metalldraht (122) der ersten Elektrode (111) am distalen Ende fortfährt, sich distal zu erstrecken, und gebogen ist, um den Fixierungshaken (113) zu bilden.

7. System zur peripheren Nervenstimulation nach Anspruch 6, **dadurch gekennzeichnet, dass** der erste Leiterdraht (1211) zwischen der ersten Elektrode (111) und der zweiten Elektrode (112) mit einer isolierenden Pufferhülse (114) versehen ist; der Biegewinkel des Fixierungshakens 20-60 Grad beträgt; der Metalldraht (122) einen Durchmesser von 0,1 mm - 0,15 mm aufweist und der Außendurchmesser der ersten Elektrode (111) und/oder der zweiten Elektrode (112) 0,55 mm - 0,70 mm beträgt; wobei der freigelegte Metalldraht (122) an die erste Elektrode (111) oder die zweite Elektrode (112) geschweißt oder gecrimpt ist.

8. System zur peripheren Nervenstimulation nach Anspruch 1, **dadurch gekennzeichnet, dass** der Metalldraht (122) ein 316L-Edelstahldraht oder ein nichtmagnetischer MP35N-Nickel-Kobalt-Chrom-Molybdän-Legierungsdraht ist; das Isoliermaterial, das für die Isolierschicht (123) verwendet wird, Polyurethan, Teflon oder Ethylen-Tetrafluorethylen-Copolymer ist und das Isoliermaterial in einer Röhrenform extrudiert und dann auf die Peripherie des Metalldrahts (122) aufgebracht wird oder das Isoliermaterial durch Verwenden eines Beschichtungsverfahrens auf den Metalldraht (122) gesprüht wird.

9. System zur peripheren Nervenstimulation nach Anspruch 1, **gekennzeichnet durch** ferner Umfassen eines Montagesitzes (21); wobei der Adapter (20) auf dem Montagesitz (21) montiert ist; der Montagesitz (21) einen Hauptkörperabschnitt (212) und eine Basis (211) umfasst; der Hauptkörperabschnitt (212) auf der Basis (211) angeordnet ist; der Hauptkörperabschnitt (212) eine gewölbte ausgehöhlte Struktur aufweist; die Bodenfläche der Basis (211) mit einem hautfreundlichen Klebstoff versehen ist und der Montagesitz (21) durch den hautfreundlichen Klebstoff an einer Wundperipherie eines Patienten anbringbar ist; wobei eine erste Führungsschiene (2111), eine zweite Führungsschiene (2112) und ein erster Druckknopf (2114) auf der Basis (211) angeordnet sind; ein erster Seitenflügel (2021) und ein zweiter Seitenflügel (2022) jeweils auf beiden Seiten des Bodens des Adapters (20) angeordnet sind und der erste Seitenflügel (2021) in die erste Führungsschiene (2111) eingesetzt wird, der zweite Seitenflügel (2022) in die zweite Führungsschiene (2112) eingesetzt wird und die Basis (211) und der Adapter (20) durch den ersten Druckknopf (2114) befestigt werden.

10. System zur peripheren Nervenstimulation nach Anspruch 9, **dadurch gekennzeichnet, dass** die Basis (211) und das Oberteil (2121) des Hauptkörperabschnitts (212) sowohl ringförmig als auch koaxial angeordnet sind; der Außendurchmesser des Oberteils (2121) des Hauptkörperabschnitts (212) kleiner als der Außendurchmesser der Basis (211) ist und eine Vielzahl von Bogenstreifen (2122) zwischen dem Oberteil (2121) und der Basis (211) verbunden ist; wobei die Anzahl von Bogenstreifen (2122) 5-8 beträgt; wobei der Adapter (20) auf der Basis (211) zwischen zwei der angrenzenden Bogenstreifen (2122) angeordnet ist; wobei die restliche Vielzahl von Bogenstreifen (2122) gleichmäßig in einer Umfangsrichtung beabstandet ist.

**11.** System zur peripheren Nervenstimulation nach Anspruch 5, **dadurch gekennzeichnet, dass** der Adapter (20) ein erstes Gehäuse (201) und ein zweites Gehäuse (202) umfasst, die zusammenpassend und lösbar verbunden sind, wobei ein erster Kanal (203) und ein zweiter Kanal (204) in dem zweiten Gehäuse (202) angeordnet ist; ein erster Biegungsstreifen (2031) und ein zweiter Biegungsstreifen (2032) in dem ersten Kanal (203) entgegengesetzt angeordnet sind; ein dritter Biegungsstreifen (2041) und ein vierter Biegungsstreifen (2042) in dem zweiten Kanal (204) entgegengesetzt vorgesehen sind; der erste Biegungsstreifen (2031) ein erstes Blatt (2033) mit einer schrägen Öffnung aufweist, der zweite Biegungsstreifen (2032) ein zweites Blatt (2034) mit einer schrägen Öffnung aufweist, der dritte Biegungsstreifen (2041) ein drittes Blatt (2043) mit einer schrägen Öffnung aufweist und der vierte Biegungsstreifen (2042) ein viertes Blatt (2044) mit einer schrägen Öffnung aufweist; das erste Blatt (2033) und das zweite Blatt (2034) einen V-förmigen konkaven Winkel zueinander bilden, das dritte Blatt (2043) und das vierte Blatt (2044) einen V-förmigen konkaven Winkel zueinander bilden; der erste Leiterdraht (1211) in den ersten Kanal (203) eintritt und an dem V-förmigen konkaven Winkel platziert wird, der von dem ersten Blatt (2033) und dem zweiten Blatt (2034) gebildet wird; der zweite Leiterdraht (1212) in den zweiten Kanal (204) eintritt und an dem V-förmigen konkaven Winkel platziert wird, der von dem dritten Blatt (2043) und dem vierten Blatt (2044) gebildet wird; wenn das erste Gehäuse (201) gegen das zweite Gehäuse (202) gedrückt wird, das erste Blatt (2033) und das zweite Blatt (2034) zusammenwirken, um die Isolierschicht (123) des ersten Leiterdrahts (1211) zu schneiden, und das dritte Blatt (2043) und das vierte Blatt (2044) zusammenwirken, um die Isolierschicht (123) des zweiten Leiterdrahts (1212) zu schneiden, um den ersten Leiterdraht (1211) und den zweiten Leiterdraht (1212) elektrisch mit dem Adapter (20) zu verbinden; und eine Schnittstelle (2023) auf dem zweiten Gehäuse (202) angeordnet ist und das erste Kabel (30) mittels der Schnittstelle (2023) mit dem ersten Kanal (203) bzw. dem zweiten Kanal (204) verbunden ist.

**12.** System zur peripheren Nervenstimulation nach Anspruch 1, **dadurch gekennzeichnet, dass** die Oberflächenelektrode eine feststehende Oberflächenelektrode (70) ist, wobei die feststehende Oberflächenelektrode (70) am Boden des Stimulators (50) angeordnet ist und die feststehende Oberflächenelektrode (70) in einer direkten elektrischen Verbindung mit dem Stimulator (50) steht; oder die Oberflächenelektrode eine bewegliche Oberflächenelektrode (80) ist, die durch das zweite Kabel (72) elektrisch mit dem Stimulator (50) verbunden ist; und die bewegliche Oberflächenelektrode (80) an einem gewünschten Teil des menschlichen Körpers platziert werden kann.

**13.** System zur peripheren Nervenstimulation nach Anspruch 1, **dadurch gekennzeichnet, dass** die Fernbedienung (60) einen Handgriff (64), eine Steuertaste (61), einen Anzeigebildschirm (62) und ein Aufhängeloch (63) umfasst, wobei die Steuertaste (61) an einem Endabschnitt des Handgriffs (64) angeordnet ist, das Aufhängeloch (63) an einem Ausläuferende des Handgriffs (64) angeordnet ist und der Anzeigebildschirm (62) an einer oberen Fläche des Handgriffs (64) angeordnet ist; oder die die Fernbedienung (60) einen Handgriff (64), eine Steuertaste (61), einen Anzeigebildschirm (62) und eine Ladeschnittstelle (65) umfasst, wobei die Steuertaste (61) und der Anzeigebildschirm (62) auf einer oberen Fläche des Handgriffs (64) angeordnet ist und die Ladeschnittstelle (65) auf einer Seitenfläche des Handgriffs (64) angeordnet ist.

**14.** System zur peripheren Nervenstimulation nach Anspruch 1, **gekennzeichnet durch** Umfassen eines klinischen Programmiergeräts (100), das mit einem Tablet-Computer implementiert ist, wobei ein Kliniker Stimulationsparameter und Stimulationsprogramme des Stimulators einstellt oder justiert und Zustände der perkutanen implantierten Leitung (10) für den Patienten während der Implantations- oder Behandlungsstufe durch eine Programmierschnittstelle des klinischen Programmiergeräts diagnostiziert.

## Revendications

**1.** Système de stimulation nerveuse périphérique, qui comprend une sonde percutanée implantée (10), un adaptateur (20), un premier câble (30), un stimulateur (50) et une électrode de surface ;

la sonde percutanée implantée (10) peut être insérée dans le corps d'un patient à travers la peau, et la sonde percutanée implantée (10) comprend une tête de sonde distale (11) et un corps de sonde (12) d'une extrémité distale à une extrémité proximale, la tête de sonde distale (11) comprenant au moins une électrode, le corps de sonde (12) étant formé par l'enroulement en spirale d'au moins un fil conducteur (121), et le fil conducteur (121) comprenant un fil métallique (122) et une couche isolante (123), qui est agencée à l'extérieur du fil métallique (122) d'une manière enveloppante, de sorte que le corps de sonde (12) soit d'une structure en spirale isolante ouverte ;
l'adaptateur (20) est électriquement connecté à l'extrémité proximale de la sonde percutanée implantée (10) ; l'adaptateur (20) est électriquement connecté au stimulateur (50) au moyen du premier câble (30) ; le stimulateur (50) est utilisé pour l'envoi d'une impulsion de stimulation électrique ; et

l'électrode de surface est électriquement connecté au stimulateur (50), et forme une boucle de stimulation conjointement avec la sonde percutanée implantée (10) à travers un tissu humain stimulé ;

**caractérisé par le fait que** le système de stimulation nerveuse périphérique comprend en outre une télécommande (60) pour un patient, la télécommande (60) étant connectée au stimulateur (50) par communication Bluetooth ; et

la télécommande (60) stocke des données ordinales de programme de stimulation et des données ordinales d'intensité de stimulation correspondant aux contenus d'une table de programme et d'une table d'intensité dans une mémoire renforcée du stimulateur (50), respectivement.

**2.** Système de stimulation nerveuse périphérique selon la revendication 1, **caractérisé en ce que** la tête de sonde distale (11) comprend une première électrode (111) et un crochet de fixation (113) ; l'au moins un fil conducteur (121) ayant un fil conducteur (121) ; les spires constituant le corps de sonde (12) sont agencées en séquence selon un pas prédéterminé dans une direction axiale ; la première électrode (111) est formée dans une direction distale dans un état exposé par l'enroulement en spirale du fil métallique (122) constituant le fil conducteur (121) ; et le fil métallique (121) à l'extrémité distale de la première électrode (111) continue de s'étendre de manière distale et est cintré pour former le crochet de fixation (113) ; le pas de la première électrode (111) entre deux spires adjacentes dans les 2 à 3 spires de l'extrémité distale de celle-ci est plus grand que le pas ailleurs.

**3.** Système de stimulation nerveuse périphérique selon la revendication 1, **caractérisé en ce que** la tête de sonde distale (11) comprend une première électrode (111) et un crochet de fixation (113) ; dans lequel la première électrode (111) est une électrode métallique tubulaire ; l'au moins un fil conducteur (121) ayant un fil conducteur (121) ; les spires constituant le corps de sonde (12) sont agencées en séquence selon un pas prédéterminé le long de la direction axiale ; le fil métallique (122) constituant le fil conducteur (121) s'étend de manière distale à travers un tube d'électrode de l'électrode métallique dans un état exposé et continue à s'étendre et à se cintrer pour former le crochet de fixation (113) ; et le fil métallique (122) est électriquement connecté à l'électrode métallique.

**4.** Système de stimulation nerveuse périphérique selon la revendication 1, **caractérisé en ce que** la tête de sonde distale (11) comprend une première électrode (111) et un crochet de fixation (113), dans lequel la première électrode (111) est une électrode métallique tubulaire ; l'au moins un fil conducteur (121) ayant un fil conducteur (121) ; les spires constituant le corps de sonde (12) sont agencées en séquence selon un pas prédéterminé le long de la direction axiale ; une extrémité distale du fil métallique (122) constituant le fil conducteur (121) s'étend et s'étend ensuite dans une extrémité proximale d'un tube d'électrode de la première électrode (111) et est électriquement connectée à la première électrode (111) ; le crochet de fixation (113) est constitué du fil métallique (122) ; et l'extrémité proximale du crochet de fixation (113) s'étend dans une extrémité distale du tube d'électrode de la première électrode (111) et est connectée à la première électrode (111).

**5.** Système de stimulation nerveuse périphérique selon la revendication 1, **caractérisé en ce que** la tête de sonde distale (11) comprend une deuxième électrode (112), une première électrode (111) et un crochet de fixation (113) en séquence de l'extrémité proximale à l'extrémité distale, dans lequel la première électrode (111) et la deuxième électrode (112) sont agencées le long d'un même intervalle axial et isolées ; l'au moins un fil conducteur (121) ayant un premier fil conducteur (1211) et un deuxième fil conducteur (1212) ; les spires constituant le corps conducteur (12) sont agencées en séquence selon un pas prédéterminé le long de la direction axiale ; la première électrode (111) est connectée à l'adaptateur (20) via le premier fil conducteur (1211) ; la deuxième électrode (112) est connectée à l'adaptateur (20) via le deuxième fil conducteur (1212) ; une électrode quelconque ou une combinaison de deux électrodes quelconques parmi la première électrode (111), la deuxième électrode (112) et l'électrode de surface étant utilisées comme électrode de stimulation ou électrode de retour, et constitue une boucle de stimulation conjointement avec les une ou deux électrodes restantes à travers un tissu humain stimulé.

**6.** Système de stimulation nerveuse périphérique selon la revendication 5, **caractérisé en ce que** la première électrode (111) et la deuxième électrode (112) sont toutes les deux des électrodes métalliques tubulaires ; le premier fil conducteur (1211) est isolé pour passer à travers le tube d'électrode de la deuxième électrode (112) ; le fil métallique (122) constituant le premier fil conducteur (1211) s'étend de manière distale à travers le tube d'électrode de la première électrode (111) et continue ensuite à s'étendre de manière distale et à se cintrer pour former le crochet de fixation (113), le fil métallique (122) du premier fil conducteur (1211) étant électriquement connecté à la première électrode (111) ; le fil métallique (122) exposé du deuxième fil conducteur (1212) à l'extrémité distale s'étend dans le tube d'électrode de la deuxième électrode (112) et est électriquement connecté à la deuxième électrode (112) ; ou, le fil métallique (122) constituant le deuxième fil conducteur (1212) s'étend de manière distale dans un état exposé et est enroulé en spirale pour former la deuxième électrode (112) ; l'extrémité distale du premier fil conducteur (1211) est isolée pour passer à travers une cavité formée en spirale par la deuxième électrode (112) ; le fil métallique (122) constituant le premier fil conducteur (1211) s'étend de manière distale dans un état exposé et est enroulé en spirale pour former la première électrode (111) ; et le fil métallique (122) de la première électrode (111) à l'extrémité distale continue à s'étendre de manière distale et est cintré pour former le crochet de fixation (113).

**7.** Système de stimulation nerveuse périphérique selon la revendication 6, **caractérisé en ce que** le premier fil conducteur (1211) entre la première électrode (111) et la deuxième électrode (112) est muni d'un manchon tampon isolant (114) ; l'angle de cintrage du crochet de fixation est de 20 à 60 degrés ; le fil métallique (122) possède un diamètre de 0,1 mm à 0,15 mm, et le diamètre externe de la première électrode (111) et/ou de la deuxième électrode (112) est de 0,55 mm à 0,70 mm ; le fil métallique (122) exposé est soudé ou serti sur la première électrode (111) ou la deuxième électrode (112).

**8.** Système de stimulation nerveuse périphérique selon la revendication 1, **caractérisé en ce que** le fil métallique (122) est un fil en acier inoxydable 316L ou un fil en alliage nickel-cobalt-chrome-molybdène non magnétique MP35N ; le matériau isolant utilisé pour la couche isolante (123) est du polyuréthane, du téflon ou un copolymère éthylène-tétrafluoroéthylène ; et le matériau isolant est extrudé en une forme tubulaire et appliqué ensuite sur la périphérie du fil métallique (122), ou le matériau isolant est pulvérisé sur le fil métallique (122) en utilisant un processus d'enduction.

**9.** Système de stimulation nerveuse périphérique selon la revendication 1, **caractérisé par le fait qu'**il comprend en outre un siège de montage (21) ; l'adaptateur (20) est monté sur le siège de montage (21) ; le siège de montage (21) comprend une portion corps principal (212) et une base (211) ; la portion corps principal (212) est disposée sur la base (211) ; la portion corps principal (212) possède une structure évidée arquée ; la surface inférieure de la base (211) est munie d'un adhésif respectueux de la peau ; et le siège de montage (21) est apte à être fixé à la périphérie d'une plaie d'un patient grâce à l'adhésif respectueux de la peau ; un premier rail de guidage (2111), un deuxième rail de guidage (2112) et un premier fermoir (2114) sont disposés sur la base (211) ; une première aile latérale (2021) et une deuxième aile latérale (2022) sont respectivement disposées sur les deux côtés du bas de l'adaptateur (20) ; et la première aile latérale (2021) est insérée dans le premier rail de guidage (2111), la deuxième aile latérale (2022) est insérée dans le deuxième rail de guidage (2112), et la base (211) et l'adaptateur (20) sont fixés par le premier fermoir (2114).

**10.** Système de stimulation nerveuse périphérique selon la revendication 9, **caractérisé en ce que** la base (211) et le haut (2121) de la portion corps principal (212) sont tous les deux disposés de manière annulaire et coaxiale ; le diamètre externe du haut (2121) de la portion corps principal (212) est inférieur au diamètre externe de la base (211) ; et une pluralité de bandes arquées (2122) sont reliées entre le haut (2121) et la base (211) ; le nombre de bandes arquées (2122) est de 5 à 8 ; l'adaptateur (20) est disposé sur la base (211) entre deux des bandes arquées (2122) adjacentes ; la pluralité restante de bandes arquées (2122) sont espacées de manière uniforme dans une direction circonférentielle.

**11.** Système de stimulation nerveuse périphérique selon la revendication 5, **caractérisé en ce que** l'adaptateur (20) comprend un premier boîtier (201) et un deuxième boîtier (202) reliés de manière accouplée et détachable, un premier canal (203) et un deuxième canal (204) étant disposés dans le deuxième boîtier (202) ; une première bande de flexion (2031) et une deuxième bande de flexion (2032) sont disposées de manière opposée dans le premier canal (203) ; une troisième bande de flexion (2041) et une quatrième bande de flexion (2042) sont ménagées de manière opposée dans le deuxième canal (204) ; la première bande de flexion (2031) possède une première lame (2033) dotée d'une ouverture oblique, la deuxième bande de flexion (2032) possède une deuxième lame (2034) dotée d'une ouverture oblique, la troisième bande de flexion (2041) possède une troisième lame (2043) dotée d'une ouverture oblique, et la quatrième bande de flexion (2042) possède une quatrième lame (2044) dotée d'une ouverture oblique ; la première lame (2033) et la deuxième lame (2034) forment un angle concave en forme de V l'une par rapport à l'autre, la troisième lame (2043) et la quatrième lame (2044) formant un angle concave en forme de V l'une par rapport à l'autre ; le premier fil conducteur (1211) passe dans le premier canal (203) et est placé à l'angle concave en forme de V formé par la première lame (2033) et la deuxième lame (2034) ; le deuxième fil conducteur (1212) passe dans le deuxième canal (204) et est placé à l'angle concave en forme de V formé par la troisième lame (2043) et la quatrième lame (2044) ; lorsque le premier boîtier (201) est pressé contre le deuxième boîtier (202), la première lame (2033) et la deuxième lame (2034) coopèrent pour couper la couche isolante (123) du premier fil conducteur (1211), et la troisième lame (2043) et la quatrième lame (2044) coopèrent pour couper la couche isolante (123) du deuxième fil conducteur (1212) pour connecter électriquement le premier fil conducteur (1211) et le deuxième fil conducteur (1212) à l'adaptateur (20) ; et une interface (2023) est disposée sur le deuxième boîtier (202), et le premier câble (30) est connecté au premier canal (203) et au deuxième canal (204), respectivement, via l'interface (2023).

12. Système de stimulation nerveuse périphérique selon la revendication 1, **caractérisé en ce que** l'électrode de surface est une électrode de surface fixe (70), l'électrode de surface fixe (70) étant disposée sur le bas du stimulateur (50), et l'électrode de surface fixe (70) étant en connexion électrique directe avec le stimulateur (50) ; ou l'électrode de surface est une électrode de surface mobile (80) qui est électriquement connectée au stimulateur (50) par le deuxième câble (72) ; et l'électrode de surface mobile (80) peut être placée à une partie souhaitée du corps humain.

13. Système de stimulation nerveuse périphérique selon la revendication 1, **caractérisé en ce que** la télécommande (60) comprend une poignée (64), une touche de commande (61), un écran d'affichage (62), et un trou pour l'accrocher (63), dans lequel la touche de commande (61) est disposée au niveau d'une portion d'extrémité de la poignée (64), le trou pour l'accrocher (63) est disposé au niveau d'une portion de queue de la poignée (64), et l'écran d'affichage (62) est disposé au niveau d'une surface de dessus de la poignée (64) ; ou, la télécommande (60) comprend une poignée (64), une touche de commande (61), un écran d'affichage (62) et une interface de charge (65), dans lequel la touche de commande (61) et l'écran d'affichage (62) sont disposés sur une surface de dessus de la poignée (64), et l'interface de charge (65) est disposée sur une surface latérale de la poignée (64).

14. Système de stimulation nerveuse périphérique selon la revendication 1, **caractérisé par le fait qu'**il comprend un programmeur clinique (100) mis en œuvre avec un ordinateur tablette, dans lequel un clinicien définit ou règle des paramètres de stimulation et des programmes de stimulation du stimulateur et diagnostique des états de la sonde percutanée implantée (10) pour le patient pendant l'étape d'implantation ou de traitement par une interface de programmation du programmeur clinique.

FIG. 1

FIG.2a

FIG. 2b

FIG. 2c

FIG. 3a

FIG. 3b

FIG. 4a

FIG. 4b

FIG. 5

FIG. 6

FIG. 7

FIG. 8a

FIG. 8b

FIG. 8c

FIG. 8d

FIG. 8e

FIG. 9

FIG. 10

（a）

(b)

FIG. 11

(a)

(b)

FIG. 12

Shared unipolar
pulse source

52

Output switch

51

VH

Amplitude

Current
source

GND

Switching

Stimulation
controller

Stimulation pulse generator

Output C

Output B

Output A

FIG. 13

(a)

(b)

FIG. 14

FIG. 15

FIG. 16a

Ramp-Up/Down
Burst

Burst
Stimulation

Constant
Continuous
Stimulation

EP 4 364 789 B1

33

Dual Frequency
Continuous
stimulation

Dual Frequency
Burst stimulation

Ramp-Up/Down
Burst stimulation

FIG. 16b

FIG. 17

FIG. 18

FIG. 19

FIG. 20

FIG. 21

| Stimulation electrode: E1+E2 | Channel electrode: E3 |
|---|---|

Waveform template

Amplitude: 5.0 **mA**

Frequency: 50 **Hz**

Pulse width: 100 **μs**

Interphase width: 50 **μs**

Polarity: **CF / AF**

Continuous modulation mode

Modulation manner: FM/AM

Modulation depth: 30 %

Burst stimulation period: 5 **S**

Duty cycle: 60 %

| Stimulation | Storage | Hardened |
|---|---|---|

(a) Programmer interface: simulation modulation mode

| Stimulation electrode: E1+E2 | Channel electrode: E3 |
|---|---|

**Waveform template**

**Amplitude: mA**

**Frequency: Hz**

**Pulse width: µs**

**Interphase width: µs**

**Polarity: CF / AF**

Keying shift regulation mode: | T1 | T2

| Keying shift period: | T1: S | T2: S |
|---|---|---|
| Keying shift frequency: | F1: Hz | F2: Hz |
| Keying shift amplitude: | A1: mA | A2: mA |
| Burst stimulation: | Cycle: S | Duty cycle: % |

| Stimulation | Storage | Hardened |
|---|---|---|

(b) Programmer interface: keying shift
modulation mode

FIG. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4556051 A
- US 5830151 A
- US 20180056066 A1
- US 2010152808 A1